(19) Europäisches Patentamt
European Patent Office
Office européen des brevets

(11) Veröffentlichungsnummer: **0 147 361**
**B1**

(12) # EUROPÄISCHE PATENTSCHRIFT

(45) Veröffentlichungstag der Patentschrift: **08.02.89**

(51) Int. Cl.⁴: **C 07 J 41/00**, C 07 J 1/00, C 07 J 31/00, C 07 J 43/00, C 07 J 51/00, C 07 J 63/00, A 61 K 31/565, A 61 K 31/58

(21) Anmeldenummer: **84730147.0**

(22) Anmeldetag: **18.12.84**

(54) **11 Beta-Aryl-Estradiene, deren Herstellung und diese enthaltende pharmazeutische Präparate.**

(30) Priorität: **22.12.83 DE 3347126**

(43) Veröffentlichungstag der Anmeldung: **03.07.85 Patentblatt 85/27**

(45) Bekanntmachung des Hinweises auf die Patenterteilung: **08.02.89 Patentblatt 89/6**

(84) Benannte Vertragsstaaten: **AT BE CH DE FR GB IT LI LU NL SE**

(56) Entgegenhaltungen:
**EP-A- 0 057 115**
**EP-A- 0 071 153**
**EP-A- 0 116 974**
**WO-A-83/03099**
**FR-A- 2 377 418**

**STEROIDS, vol. 37, no. 4, April 1981, Seiten 361-382, San Fransico, US; A. BELANGER et al.: "Regio and stereospecific synthesis of 11-alpha-substitud 19-norsteroids"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Rohde, Ralph, Dr., Schwatlowstrasse 4, D-1000 Berlin 45 (DE)**
Erfinder: **Annen, Klaus, Dr., Osthofstrasse 80, D-4400 Münster-Albachten (DE)**
Erfinder: **Neef, Günter, Dr., Darmstädter Strasse 9, D-1000 Berlin 15 (DE)**
Erfinder: **Wiechert, Rudolf, Prof., Dr., Petzower Strasse 8 A, D-1000 Berlin 39 (DE)**
Erfinder: **Beier, Sybille, Dr., Uhlandstrasse 121, D-1000 Berlin 31 (DE)**
Erfinder: **Elger, Walter, Dr., Schorlemer Allee 12 B, D-1000 Berlin 33 (DE)**
Erfinder: **Henderson, David, Dr., Jahnstrasse 17, D-1000 Berlin 28 (DE)**

**EP 0 147 361 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 11β-Aryl-Estradiene der allgemeinen Formel I

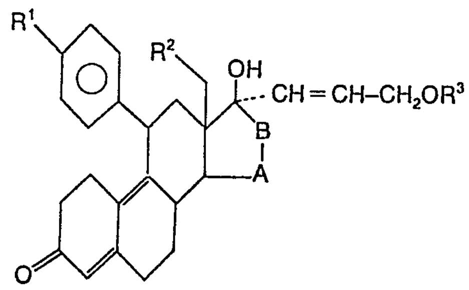

(I),

Verfahren zu deren Herstellung und diese Verbindungen enthaltende pharmazeutische Präparate.

11β-Aryl-Steroide sind bereits bekannt. So werden beispielsweise 11β-Aryl-17α-propinyl- und -ethinyl-4,9(10)-estradiene in der EP-A-0 057 115 und der US-Patentschrift 4 386 085 als Verbindungen mit antigestagenen und antiglucocorticoiden Eigenschaften beschrieben.

Dieser Stand der Technik wird durch «Steroids 37 (1981), 361–382» sowie durch die Druckschrift WO-A-8 303 099 ergänzt insoweit, als sie gleichartige Wirkungen wie in EP-A-0 057 115 beschrieben, auch für Verbindungen offenbaren, in denen der 11β-Phenylrest in 4-Stellung methoxy- bzw. thiomethyl-substituiert ist. Aus «Steroids 37 (1981), 361–382» gehen 11β-(4-Methoxy)-aryl-substituierte

17α-Ethinyl-17β-hydroxy-4,9-estradien-3-one

hervor; WO-A-8 303 099 beschreibt das entsprechende

11β-4-Methylthiophenyl-17β-hydroxy-17α-(prop-1-inyl)estra-4,9-dien-3-on,

die entsprechenden 17α-prop-2-inyl- und -prop-2-enyl-Verbindungen sowie die analogen 4-Methoxyphenyl-Derivate.

In FR-A-2 377 418 ist das

11β-(p-Methoxyphenyl)-17α-ethinyl-17-hydroxy-estra-4,9-dien-3-on

beschrieben, das antigestagene Wirkung besitzt.

Die antigestagene Wirksamkeit aller dieser Verbindungen, besonders in sehr niederen Dosen, darf als gering bezeichnet werden.

Androstanderivate, die in 11β-Stellung keinen Phenylsubstituenten tragen, aber als 17α-Substituenten mit einer 3-Acyloxyprop-1-enyl-Gruppe substituiert sein können, sind in der EP-A-0 071 153 erwähnt. Eine antigestagene Wirkung zeigen die in der EP-A-0 071 153 beschriebenen Verbindungen nicht.

Es wurde nun gefunden, dass die neuen Verbindungen der allgemeinen Formel I

worin

$R^1$ 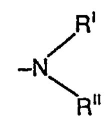

mit $R^I$ und $R^{II}$ in der Bedeutung von Wasserstoff, Alkyl mit 1 bis 4 Kohlenstoffatomen oder Acyl mit 1 bis 8 Kohlenstoffatomen oder $R^I$ und $R^{II}$ unter Einschluss von N in der Bedeutung eines 5- oder 6gliedrigen Ringes, wobei im Ring ausser N noch ein weiteres Heteroatom wie O, N, S enthalten sein kann, sowie die entsprechenden N-Oxide und Säureadditionssalze oder $R^I$ und $R^{II}$ unter Einschluss von N in der Bedeutung eines gegebenenfalls durch Methyl substituierten Pyrrolringes,

$-OR^{III}$ mit $R^{III}$ in der Bedeutung von Wasserstoff, Methyl, Ethyl, Propyl, Methoxyphenyl, Allyl oder Dimethylaminoethyl,

$-SR^{IV}$ mit $R^{IV}$ in der Bedeutung von Wasserstoff, Methyl, Ethyl, Propyl oder Dimethylaminoethyl,

$R^2$ ein Wasserstoffatom, eine Methyl- oder Ethylgruppe,

$R^3$ ein Wasserstoffatom oder eine Acylgruppe mit 1 bis 8 Kohlenstoffatomen und

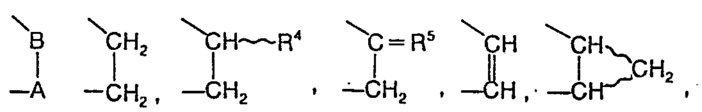

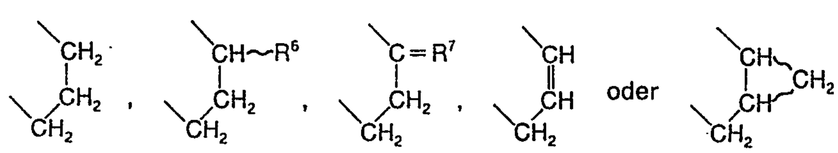

bedeuten, wobei $R^4$ und $R^6$ jeweils Alkyl mit 1 bis 4 Kohlenstoffatomen in α- oder β-Stellung und $R^5$ und $R^7$ jeweils eine E- oder Z-konfigurierte Alkylidengruppe darstellen und die $C_{20}/C_{21}$-Doppelbindung eine Z- oder E-Konfiguration besitzt, antigestagene und antimineralcorticoide Wirkungen besitzen. Das gleichzeitige Auftreten von antigestagener und antimineralcorticoider Wirkung bei einer Substanz ist bisher noch nicht beschrieben worden. Überraschenderweise treten die beiden Effekte bei den neuen Verbindungen auch bei oraler Applikation in einer gegenüber den Verbindungen des Standes der Technik vergleichbaren Wirkungsstärke auf, wobei bei einigen erfindungsgemässen Verbindungen die antigestagene und bei anderen die antimineralcorticoide Wirkung überwiegt. Die neuen Verbindungen der allgemeinen Formel I sind damit prinzipiell sowohl zur Fertilitätskontrolle als auch zur Behandlung von Krankheitszuständen geeignet, an denen ein Hyperaldosteronismus beteiligt ist.

Die bei den Verbindungen des Standes der

Technik gefundene antiglucocorticoide Wirkung tritt bei den erfindungsgemässen Verbindungen nicht mehr auf.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 pc.).

Die Behandlung der Tiere mit der jeweils zu testenden Substanz bzw. dem Lösungsmittel erfolgte nach der Nidation der Blastocysten von d5 p.c. bis d7 p.c. An d9 p.c. wurden die Tiere getötet und die Uteri auf Implantate und Resorptionsstellen hin untersucht. Von allen Uteri wurden Fotos angefertigt. Das Fehlen von Implantaten wurde als Abort gewertet.

Die Testsubstanzen wurden in einem Benzylbenzoat-Rizinusöl-Gemisch (Verhältnis 1+9) gelöst. Das Vehikelvolumen pro Einzeldosis betrug 0,2 ml. Die Behandlung erfolgte subcutan.

Die Überlegenheit der erfindungsgemässen Verbindungen soll durch Vergleich der biologischen Eigenschaften von
11β-(4-Dimethylaminophenyl)-17β-hydroxy-17α-(3-hydroxy-prop-1(Z)-enyl)-4,9(10)-estradien-3-on
(A), dem in der EP-A-0 057 115 beschriebenen
11β-(4-Dimethylaminophenyl)-17β-hydroxy-17α-(propin-1-yl)-4,9(10)-estradien-3-on
(B), 11β-(4-Dimethylaminophenyl)-17β-hydroxy-17α-(3-hydroxyprop-1(Z)-enyl)-16β-methyl-4,9(10)-estradien-3-on
(C), 17β-Hydroxy-17α-(3-hydroxyprop-1(Z)-enyl-11β-(4-methylaminophenyl)-4,9(10)-estradien-3-on
(D) und 11β-(4-Dimethylaminophenyl)-17aβ-hydroxy-17aα-(3-hydroxyprop-1(Z)-enyl)-D-homo-4,9,16-estratrien-3-on
(E) gezeigt werden:

Tabelle 1

Abortivtest bei der graviden Ratte

| Substanz | Dosis mg/Tier/Tag s.c. | n Abort-positiv / n Gesamt |
|---|---|---|
| A | 10,0 | 4/4 |
| | 3,0 | 4/4 |
| | 1,0 | 4/4 |
| B | 10,0 | 4/4 |
| | 1,0 | 2/4 |
| C | 3,0 | 4/4 |
| | 1,0 | 4/4 |
| D | 3,0 | 4/4 |
| | 1,0 | 4/4 |
| E | 3,0 | 4/4 |
| | 1,0 | 4/4 |

Aus Tabelle 1 ist zu entnehmen, dass nur die erfindungsgemässen Verbindungen A, C, D und E bei einer Dosis von 1,0 mg abortiv voll wirksam sind.

Zur Kennzeichnung der glucocorticoiden bzw. antiglucocorticoiden Wirkung wurde der Einfluss der erfindungsgemässen Substanzen auf die DNS-Synthese in Maus-Lymphomzellen bestimmt. Das Prinzip basiert auf der Glucocorticoid-Empfindlichkeit von T-Lymphozyten; diese Empfindlichkeit bleibt bei dem davon abstammenden Lymphom-Stamm S 49.1 erhalten und lässt sich durch Einbau von $^3$H-Thymidin leicht quantitativ bestimmen. Nach Zusatz von Cortisol zu den Kulturen zeigen die Zellen eine dosisabhängige Hemmung der DNS-Synthese (s. Figur 1), wobei eine 50%ige Hemmwirkung mit ca. $10^{-6}$ M Cortisol erreicht wird. Damit potentielle agonistische und antagonistische Effekte der Testsubstanzen in Erscheinung treten können, wurden die Zellen gleichzeitig mit $10^{-6}$ M Cortisol und geeigneten Mengen der Testsubstanz behandelt (Cortisol und die Testsubstanzen wurden in Ethanol gelöst. Die Endkonzentration in Ethanol im Ansatz betrug 1%. Den Kontrollproben wurde nur Ethanol zugegeben).

Das bekannte Antiglucocorticoid B zeigt eine dosisabhängige Aufhebung der Cortisol-Hemmwirkung – eine äquimolare Konzentration ist in der Lage, die Wirkung von $10^{-6}$ M Cortisol zu neutralisieren (s. Figur 2). Die erfindungsgemässe Verbindung A zeigt dagegen keine messbare Antiglucocorticoid-Wirkung (s. Figur 2). Verbindung B, allein als Zusatz zu den Kulturen gegeben, zeigt keine agonistische (Corticoid-ähnliche) Wirkung (s. Figur 1). Verbindung A hat eine leichte Eigenwirkung, die allerdings nur 1% der Wirkung von Cortisol beträgt (s. Figur 2).

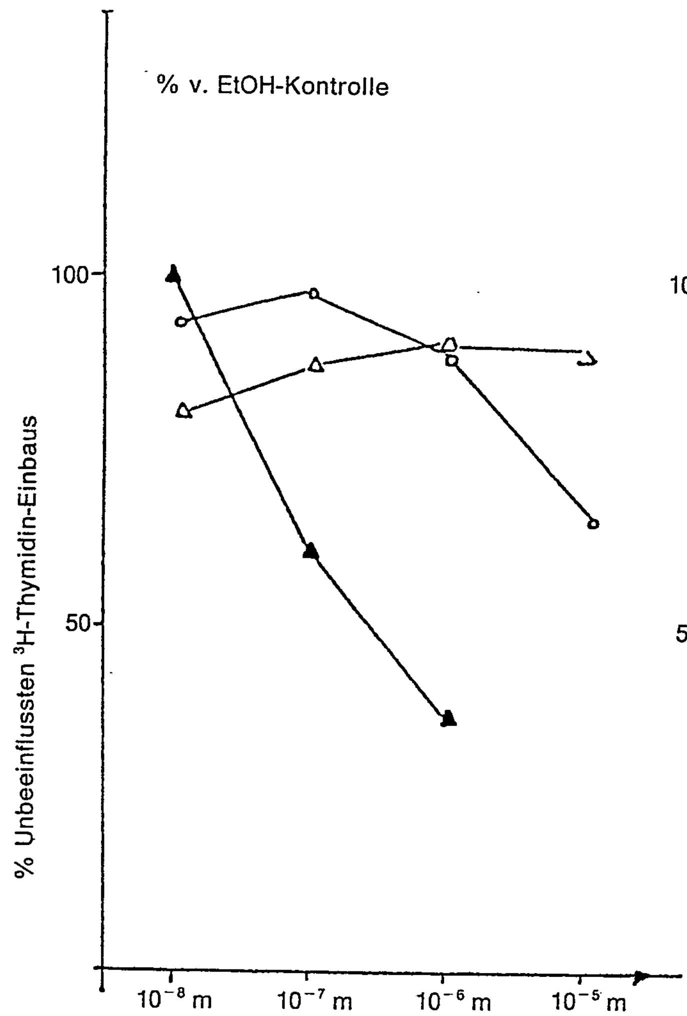


Fig. 1

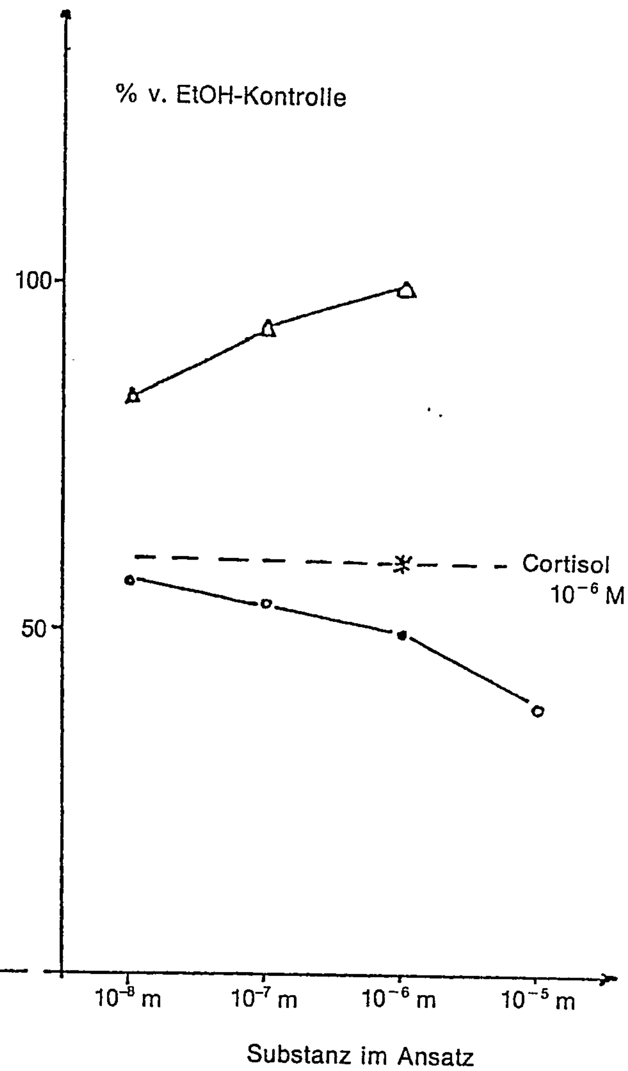


Fig. 2

Fig. 1
Eigenwirkung von Testsubstanzen auf S 49.1 Zellen
▲ Cortisol
△ B
○ A

Fig. 2
Test auf Antiglucocorticoidwirkung
△ B
○ A
Die gebrochene Linie zeigt die Hemmwirkung von $10^{-6}$ M Cortisol.

Zur Bewertung der antimineralcorticoiden Wirksamkeit der erfindungsgemässen Verbindungen dient der Antialdosterontest. In diesem Test wurden adrenalektomierte, mit Fluocortolon und Fluocortoloncapronat substituierte, nüchterne Wistar-Ratten mit 1,0–2,0–4,0 mg Prüfsubstanz pro Tier behandelt. Die Prüfsubstanz wurde als Kristallsuspension in NaCl/Myrj 53 oral appliziert. Eine Stunde nach der Applikation erhielten die Tiere eine intravenöse Dauerinfusion von physiologischer Kochsalzlösung mit einem Zusatz von 0,15 µg Aldosteron pro Tier pro Stunde. Von der dritten bis zur zehnten Stunde wurden stündlich Na- und K-Salzausscheidung gemessen, und es wurden der Na/K-Quotient sowie der Quotient log Na (100)/K ermittelt.

Es zeigte sich, dass die antimineralcorticoide Wirkung der erfindungsgemässen Verbindungen in der gleichen Grössenordnung liegt wie von Spironolakton.

Im Vergleich mit den Verbindungen des Standes der Technik besitzen die erfindungsgemässen Verbindungen stärkere antigestagene und nicht antiglucocorticoide, sondern antimineralcorticoide Wirksamkeit.

Als Antigestagene sind die erfindungsgemässen Verbindungen zur Aufhebung einer Schwangerschaft bzw. zur Auslösung einer Menstruation geeignet. Die dabei auftretende antimineralcorticoide Wirkung ist nicht schädlich und bei vorliegendem Hyperaldosteronismus sogar erwünscht.

Die Behandlung von bestimmten Formen des Aldosteronismus, der Hypertonie, von Ödemen und anderer durch Aldosteron bedingter Störungen kann auch das Hauptanwendungsgebiet der Verbindungen der allgemeinen Formel I sein.

Die Erfindung betrifft auch pharmazeutische Präparate, die Verbindungen der allgemeinen Formel I enthalten.

Die pharmakologisch wirksamen erfindungsgemässen Verbindungen der allgemeinen Formel I können nach an sich bekannten Methoden der Galenik zu pharmazeutischen Präparaten für die orale oder parenterale Applikation verarbeitet werden.

Die Dosierung der erfindungsgemässen Verbindungen liegt beim Menschen bei 10 bis 1000 mg pro Tag.

Die neuen 11β-Aryl-Estradiene enthalten in 17α-Stellung des Steroidgerüstes eine 3-Hydroxy- bzw. Acyloxyprop-1-enyl-Seitenkette mit Z- bzw. E-konfigurierter Doppelbindung. Unter Acyl sollen Carbonsäurereste mit 1 bis 8 Kohlenstoffatomen verstanden werden. Bevorzugt sind Alkanoylgruppen, wie die Acetyl-, Propionyl-, Butyryl-, Pentanoyl-, Hexanoyl- und Heptanoylgruppe.

In den Verbindungen der allgemeinen Formel I stellt der Arylrest in 11β-Stellung des Steroidgerüstes einen durch $R_1$ in para-Stellung substituierten Phenylrest dar. Wenn $R_1$ für

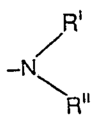

steht, bedeuten R' und R'' Alkylgruppen mit 1 bis 4 Kohlenstoffatomen, wobei die Methyl- und die Ethylgruppe bevorzugt sind. Die Gruppe

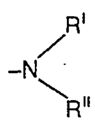

steht auch für einen heterocyclischen Fünf- oder Sechsring, der ausser N- und C-Atome auch noch zusätzlich ein O- oder S-Atom enthalten kann; beispielsweise genannt seien der Pyrrolidino-, Piperidino-, Piperazino-, Morpholino-, Oxa- und Thiazolidino- sowie Thiadiazolidinoring. Unter

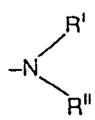

sollen auch die entsprechenden N-Oxide, wie zum Beispiel Dimethylamino-N-Oxid, Pyrrolidino-, Piperidino-, Piperazino- usw. N-Oxid und Säureadditionssalze verstanden werden. Unter

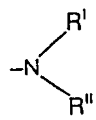

soll auch ein gegebenenfalls durch Methyl substituierter Pyrrolring, wie zum Beispiel 2,5-Dimethylpyrrol, verstanden werden.

Der Dimethylamino- und der Formylamino-Rest sind die bevorzugten Gruppen für $R_1$.

Der in Formel I durch $R^4$ und $R^6$ dargestellte Substituent ist ein Alkylrest mit 1 bis 4 Kohlenstoffatomen, vorzugsweise der Methyl- oder Ethylrest, $R^5$ und $R^7$ stellen einen $C_1$–$C_4$-Alkylidenrest, vorzugsweise den Ethylidenrest, dar.

Die neuen Verbindungen der allgemeinen Formel I werden erfindungsgemäss hergestellt, indem man in einer Verbindung der allgemeinen Formel II

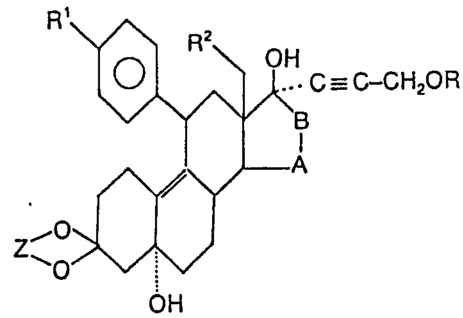

(II),

$$\cdots CH=CH-CH_2OR \quad \text{(III)},$$

worin

$R^1$, $R^2$ und

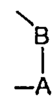

die in Formel I angegebene Bedeutung haben,

Z eine Ethylen- oder 2,2-Dimethyltrimethylengruppe und

R ein Wasserstoffatom oder eine im sauren Milieu oder hydrogenolytisch leicht abspaltbare organische Gruppe darstellt,

in Gegenwart eines desaktivierten Edelmetallkatalysators die acetylenische Dreifachbindung zur Z-konfigurierten $C_{20}/C_{21}$-Doppelbindung hydriert oder in an sich bekannter Weise die acetylenische Dreifachbindung zur E-konfigurierten $C_{20}/C_{21}$-Doppelbindung reduziert und anschliessend durch Einwirkung von verdünnter Säure, eines Pyridiniumsalzes einer starken Säure oder eines sauren Ionenaustauschers die Spaltung des 3-Ketalschutzes, die Entfernung einer gegebenenfalls vorhandenen mit Säure abspaltbaren Schutzgruppe R und die Wasserabspaltung unter Ausbildung des 4,9(10)-Dien-3-on-Systems bewirkt, und gegebenenfalls anschliessend eine freie 22-Hydroxygruppe acyliert.

In einem ersten Schritt wird die Dreifachbindung (II) der 17α-(3-oxygenierten 1-Propinyl)-Verbindung zur Z- oder E-konfigurierten Doppelbindung (III) hydriert bzw. reduziert.

Die Verbindung mit der Z-konfigurierten Doppelbindung entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134, und H.O. House: Modern Synthetic Reactions 1972, Seite 19). Als desaktivierte Edelmetallkatalysatoren kommen beispielsweise 10% Palladium auf Bariumsulfat in Gegenwart eines Amins oder 5% Palladium auf Calciumcarbonat unter Zusatz von Blei(II)-acetat infrage. Die Hydrierung wird nach

der Aufnahme von einem Äquivalent Wasserstoff abgebrochen.

Die Verbindung mit der E-konfigurierten Doppelbindung entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. Am. Chem. Soc. 77 (1955) 3378) mit Boranen (J. Am. Chem. Soc. 93 (1971) 3395 und 94 (1971) 6560), mit Diisobutyl-aluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245).

Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxidationsstufe.

Zur Wasserabspaltung unter Ausbildung des 4,9(10)-Dien-3-on-Systems und zur gleichzeitigen Ketalspaltung und Entfernung einer gegebenenfalls in 22-Stellung vorhandenen Hydroxyschutzgruppe R wird anschliessend die 17α-Oxypropenyl-17β-hydroxy-Verbindung der allgemeinen Formel III mit Säure, einem Pyridiniumsalz oder einem sauren Ionenaustauscher behandelt.

Die in der allgemeinen Formel II bzw. III durch R gekennzeichnete Hydroxyschutzgruppe ist eine im sauren Milieu oder hydrogenolytisch leicht abspaltbare Gruppe, wie zum Beispiel Methoxymethyl, Ethoxymethyl, Tetrahydropyranyl oder Benzyl.

Die saure Behandlung erfolgt in an sich bekannter Weise, indem man die Verbindung der Formel III, die eine 3-Ketalgruppe und eine 5α-Hydroxygruppe und eine gegebenenfalls O-geschützte 17α-(3-hydroxypropenyl)-Gruppe enthält, in einem mit Wasser mischbaren Lösungsmittel, wie wässrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure oder eine organische Säure, wie Essigsäure, so lange einwirken lässt, bis Wasser abgespalten ist und Schutzgruppen entfernt sind. Die Umsetzung, die bei Temperaturen von 0 bis 100 °C abläuft, kann in besonders hoher Ausbeute mit einem Pyridiniumsalz wie Pyridiniumtosylat oder einem sauren Ionenaustauscher vorgenommen werden. Der Verlauf der Umsetzung wird mit analytischen Methoden, beispielsweise durch Dünnschichtchromatographie entnommener Proben, verfolgt.

Die Acylierung der 22-Hydroxygruppe erfolgt in an sich bekannter Weise, beispielsweise durch Umsetzung mit dem Säureanhydrid in Pyridin bei Raumtemperatur.

Die Herstellung der Ausgangsverbindungen der allgemeinen Formel II soll anhand des folgenden Reaktionsschemas erläutert werden:

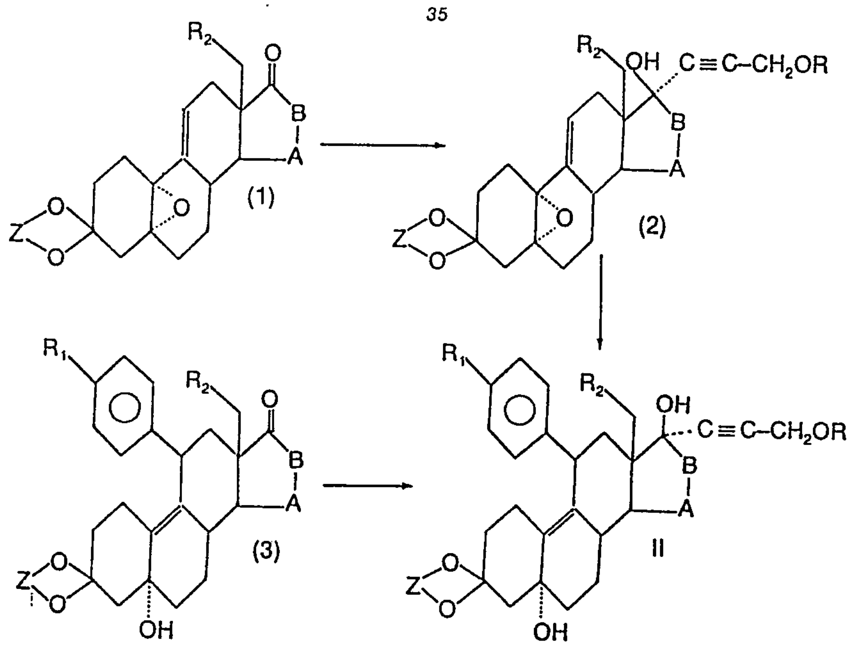
(1) (2) (3) II

Oxirane (1) gemäss europäischer Patentanmeldung Nr. 82 400 025.1 (Publikations-Nr. 0 057 115) werden mit metallierten Derivaten des Propargylalkohols, zum Beispiel mit 1-Lithium-3-tetrahydropyran-2′-yloxypropin-1, zu den 17α-[3-oxygenierten 1-propinyl]-17β-hydroxy-Verbindungen (2) umgesetzt. Die Einführung des 11β-Arylrestes zu (II) erfolgt entweder durch Cu(I)-katalysierte Grignard-Reaktion mit den entsprechenden Arylmagnesiumhalogeniden (Tetrahedron Letters 1979, 2051) oder durch Umsetzung mit gemischten Organocupraten des Typs $R_2Cu(CN)Li_2$ (J. Am. Chem. Soc. 103 (1981) 7672). Schliesslich lässt

sich das Keton (3) gemäss EP 57 115 mit freiem Propargylalkohol in Gegenwart von Basen wie Kaliumethylat, Kalium-tert.-butylat oder Butyllithium (BuLi) zu (II) umsetzen.

Im folgenden wird die Herstellung einiger Ausgangsverbindungen der allgemeinen Formel II näher erläutert:

1a) Zu einer Lösung von 35,7 g 3-Tetrahydropyran-2'yloxy-1-propin in 760 ml absolutem Tetrahydrofuran tropft man unter Eiswasserkühlung 208 ml einer 15%igen Lösung von n-Butyllithium in Hexan. Nach Zugabe rührt man weitere 15 Minuten bei +5 bis +10 °C und gibt anschliessend tropfenweise eine Lösung von 23,7 g
3,3-(2,2-Dimethyl-propan-1,3-dioxy)-5α,10α-oxido-9(11)-estren-17-on
in 470 ml absolutem THF hinzu. Man rührt danach 20 Minuten bei 25 °C, giesst daraufhin die Reaktionslösung in ca. 5 l Eiswasser und extrahiert mit Essigester. Der Essigesterextrakt wird über Natriumsulfat/Aktivkohle getrocknet und im Vakuum eingeengt. Nach Filtration über Aluminiumoxid mit Hexan/Essigester als Elutionsmitteln erhält man 29,3 g
3,3-(2,2-Dimethyl-propan-1,3-dioxy)-5α,10α-oxido-17α-[3-(tetrahydropyran-2-yloxy)-1-propinyl]-9(11)-estren-17β-ol
als farbloses Öl.

b) Eine Suspension von 5,28 g Magnesium (Späne) in 275 ml absolutem THF wird sukzessive mit 0,05 ml Methyliodid und einer Lösung von 50,27 g 4-Bromdimethylanilin in 245 ml absolutem THF versetzt. Man rührt bis zur vollständigen Auflösung des Magnesiums in einer Argon-Atmosphäre, wobei die Innentemperatur 50 °C nicht übersteigen soll. Anschliessend kühlt man auf +5 °C, versetzt die Grignard-Lösung mit 1,12 g CuCl und rührt 15 Minuten bei +5–+10 °C. Im Anschluss daran wird eine Lösung von 29,3 g des unter a) erhaltenen Produkts in 275 ml absolutem THF hinzugetropft und 5 Stunden bei Raumtemperatur nachgerührt. Dann wird die Reaktionslösung in ca. 4 l Eiswasser gegossen und mit Essigester extrahiert. Die Chromatographie des so erhaltenen Rohprodukts über Aluminiumoxid mit Hexan/Essigester ergibt 32,6 g
11β-(4-Dimethylaminophenyl)-3,3-(2,2-dimethyl-propan-1,3-dioxy)-17α-[3-(tetrahydropyran-2-yloxy)-1-propinyl]-9(10)-estren-5α,17β-diol
als gelbliches Öl.

2a) Gemäss 1a) setzt man 22,6 g
3,3-(2,2-Dimethylpropan-1,3-dioxy)-18-methyl-5α,10α-oxido-9(10)-estren-17-on
(Schmelzpunkt 156–158 °C) mit
1-Lithio-3-(tetrahydropyran-2-yloxy)-propin-1
um und erhält 25,4 g
3,3-(2,2-Dimethylpropan-1,3-dioxy)-18-methyl-5α,10α-oxido-17α-[3-(tetrahydropyran-2-yloxy)-1-propinyl]-9(11)-estren-17β-ol
als farbloses Öl.

b) Zu einer Suspension von 4,3 g Magnesium (Späne) in 40 ml absolutem THF tropft man bei 25 °C zunächst 0,1 ml Methyliodid, dann eine Lösung von 40 g p-Dimethylaminoethoxy-phenylbromid (hergestellt nach D. Lednicer et al., J. Med. Chem. 8, 52 (1964)) in 200 ml absolutem THF. Man rührt bis zur vollständigen Auflösung des Magnesiums bei einer Badtemperatur von maximal 70 °C. Nach dem Abkühlen auf 0 °C gibt man 820 mg CuCl hinzu und rührt 20 Minuten bei 0 °C. Sodann wird eine Lösung von 15,9 g des unter a) erhaltenen Produkts in 120 ml absolutem THF hinzugetropft. Man rührt 16 Stunden bei 25 °C, giesst in Eiswasser und extrahiert mit Essigester. Die Chromatographie über Aluminiumoxid (neutral, III) mit Hexan/Essigester ergibt 17,1 g
11β-(4-Dimethylaminophenyl)-3,3-(2,2-dimethylpropan-1,3-dioxy)-18-methyl-17α-[3-(tetrahydropyran-2-yloxy)-1-propinyl]-9(10)-estren-5α,17β-diol
als gelbliches Öl.

3.) Aus 2,4 g Magnesium in 120 ml absolutem THF und 11,6 ml 4-Bromanisol wird wie unter 1b) beschrieben ein Grignard-Reagenz hergestellt und mit 260 mg CuCl versetzt. Eine Lösung von 6,4 g des unter 1a) hergestellten Addukts in 80 ml absolutem THF wird bei 0 °C hinzugetropft. Die Reaktionslösung wird 4 Stunden bei 25 °C gerührt und wie unter 1b) aufgearbeitet. Man erhält 7,15 g
3,3-(2,2-Dimethylpropan-1,3-dioxy)-11β-(4-methoxyphenyl)-17α-[3-tetrahydropyran-2-yloxy)-1-propinyl]-9(10)-estren-5α,17β-diol
als öliges Produkt.

4a) Aus 6,7 g Propargyl-tetrahydropyranyläther in 100 ml THF und 40 ml n-Butyllithium (15% in Hexan) wird unter den Bedingungen von 1a) die lithiumorganische Verbindung hergestellt und mit 4,63 g
3,3-(2,2-Dimethyl-propan-1,3-dioxy)-16β-methyl-5α,10α-oxido-9(11)-estren-17-on
umgesetzt. Nach Chromatographie erhält man 4,22 g
3,3-(2,2-Dimethyl-propan-1,3-dioxy)-16β-methyl-5α,10α-oxido-17α-[3-(tetrahydropyran-2-yloxy)-prop-1-inyl]-9(11)-estren-17β-ol
als kristallines Isomerengemisch vom Schmelzpunkt 156–166 °C.

b) Aus 1,23 g Magnesiumspänen in 100 ml absolutem THF, 11,48 g 4-Dimethylaminophenylbromid in 50 ml absolutem THF, 0,03 ml Methyliodid und 230 mg CuCl wird unter den Bedingungen von 1b) ein Grignard-Reagenz hergestellt und mit 3,55 g
3,3-(2,2-Dimethyl-propan-1,3-dioxy)-16β-methyl-5α,10α-oxido-17α-(3-tetrahydropyran-2-yloxy-prop-1-inyl)-9(11)-estren-17β-ol
umgesetzt. Man erhält nach Chromatographie 3,56 g
11β-(4-Dimethylaminophenyl)-3,3-(2,2-dimethyl-propan-1,3-dioxy-16β-methyl-17α-[3-(tetrahydropyran-2-yloxy)-prop-1-inyl]-9(10)-estren-5α,17β-diol
als öliges Isomerengemisch.

5.) Zu einer Suspension von 1,02 g Magnesiumspänen und 0,05 ml Methyliodid in 25 ml absolutem Tetrahydrofuran wird eine Lösung von 10,5 g 4-(2,5-Dimethylpyrrol-1-yl) brombenzol (hergestellt nach J. Chem. Soc. 1951, 3155) in 40 ml absolutem Tetrahydrofuran in der Weise getropft, dass die Temperatur nach Anspringen der Reaktion 45 °C nicht überschreitet. Nach Auflösung des Magnesiums kühlt man auf 0 °C, gibt 210 mg CuCl hinzu, rührt 15 Minuten bei 0 °C und tropft schliesslich eine Lösung von 4,00 g

3,3-(2,2-Dimethylpropan-1,3-dioxy)-5α,10α-oxido-17α-[3-(tetrahydropyran-2-ylox)-prop-1-inyl]-9(11)-estren-17β-ol

in 50 ml absolutem Tetrahydrofuran zu. Anschliessend lässt man über Nacht bei Raumtemperatur rühren, giesst das Reaktionsgemisch auf Eiswasser und extrahiert mit Essigester. Durch Chromatographie an $Al_2O_3$ (neutral, III) mit Hexan/Essigester erhält man 4,42 g

3,3-(2,2-Dimethylpropan-1,3-dioxy)-11β-[4-(2,5-Dimethylpyrrol-1-yl)phenyl]-17α-[3-(tetrahydropyran-2-yloxy)-prop-1-inyl]-9(10)-estren-5α,17β-diol

als blassgelben festen Schaum.

6.) 1,46 g Magnesiumspäne und 0,05 ml Methyliodid werden in 15 ml absolutem Tetrahydrofuran vorgelegt. Anschliessend tropft man eine Lösung von 14,5 g N-(4-Bromphenyl)piperidin (hergestellt nach J. Am. Chem. Soc. 75, 5280 (1953)) in 100 ml absolutem Tetrahydrofuran in der Weise zu, dass die Temperatur nach Anspringen der Reaktion 45 °C nicht überschreitet. Nach Auflösung des Magnesiums kühlt man auf 0 °C, gibt 450 mg CuCl hinzu, rührt 15 Minuten bei 0 °C und tropft schliesslich eine Lösung von 6,0 g

3,3-(2,2-Dimethylpropan-1,3-dioxy)-5α,10α-oxido-17α-[3-(tetrahydropyran-2-yloxy)prop-1-inyl]-9(11)-estren-17β-ol

in 50 ml absolutem Tetrahydrofuran zu. Anschliessend lässt man über Nacht bei Raumtemperatur rühren, giesst das Reaktionsgemisch auf Eiswasser und extrahiert mit Essigester. Durch Chromatographie an $Al_2O_3$ (neutral, III) mit Hexan/Essigester erhält man 7,0 g

3,3-(2,2-Dimethylpropan-1,3-dioxy)-11β-(4-piperidinophenyl)-17α-[3-(tetrahydropyran-2-yloxy)-prop-1-inyl]-9(10)-estren-5α,17β-diol

als farblosen festen Schaum.

7.) 2,16 g Magnesiumspäne und 0,05 ml Methyliodid werden in 15 ml absolutem Tetrahydrofuran vorgelegt. Anschliessend tropft man eine Lösung von 13,5 g N-(4-Bromphenyl)pyrrolidin (hergestellt nach J. Am. Chem. Soc. 75, 5280 (1953)) in 150 ml absolutem Tetrahydrofuran in der Weise zu, dass die Temperatur des Reaktionsgemisches nach Anspringen der Reaktion 45 °C nicht überschreitet. Nach Auflösung des Magnesiums kühlt man auf 0 °C, gibt 450 mg CuCl hinzu, rührt 15 Minuten bei 0 °C und tropft schliesslich eine Lösung von 6,0 g

3,3-(2,2-Dimethyl-propan-1,3-dioxy)-5α,10α-oxido-17α-[3-(tetrahydropyran-2-yloxy)-prop-1-inyl]-9(11)-estren-17β-ol

in 70 ml absolutem Tetrahydrofuran zu. Anschliessend lässt man über Nacht bei Raumtemperatur rühren, giesst das Reaktionsgemisch auf Eiswasser und extrahiert mit Essigester. Durch Chromatographie an $Al_2O_3$ (neutral, III) mit Hexan/Essigester erhält man so 7,0 g

3,3-(2,2-Dimethyl-propan-1,3-dioxy)-11β-[4-(pyrrolidin-1-yl)phenyl]-17α-[3-(tetrahydropyran-2-yloxy)prop-1-inyl]-9(10)-estren-5α,17β-diol

als farblosen festen Schaum.

8.) Eine Lösung von 2,4 g

11β-(4-Dimethylaminophenyl)-3,3-ethylendioxy-5α-hydroxy-9-estren-17-on

(Europäische Patentanmeldung Veröffentlichungsnummer 57 115 (82)) in 36 ml wasserfreiem Tetrahydrofuran wird bei 0 °C mit 9,35 g Kaliumethylat versetzt. Man lässt innerhalb von 20 Minuten eine Lösung von 3,6 ml Propargylalkohol in 3,6 ml Tetrahydrofuran zutropfen und rührt 2 Stunden bei Raumtemperatur unter Argon weiter. Die Reaktionslösung wird auf 0 °C abgekühlt und mit ca. 8 ml eiskalter 30%iger Schwefelsäure bis zur schwach sauren Reaktion versetzt. Dabei darf die Innentemperatur des Kolbens 20 °C nicht übersteigen. Schliesslich wird die Lösung mit Natriumbicarbonatlösung neutralisiert und auf Wasser gegeben. Man extrahiert dreimal mit Essigester, wäscht die Extrakte neutral, trocknet über Natriumsulfat und engt im Vakuum zur Trockne ein. Das Rohprodukt wird an 300 g Kieselgel mit einem Methylenchlorid-Aceton-Gradienten (0–25% Aceton) chromatographiert. Ausbeute: 1,4 g

11β-(4-Dimethylaminophenyl)-3,3-ethylendioxy-17α-(3-hydroxy-1-propinyl)-9-estren-5α,17β-diol

vom Schmelzpunkt 130–132 °C.

$[\alpha]^{25}_D = -44°$.

Beispiel 1

11β-(4-Dimethylaminophenyl)-17β-hydroxy-17α-(3-hydroxyprop-1(Z)-enyl)-4,9(10)-estradien-3-on

a) Eine Lösung von 2,23 g (3,5 mmol)

3,3-(2,2-Dimethylpropan-1,3-dioxy)-11β-(4-dimethylaminophenyl)-17α-[3-(tetrahydropyran-2-yloxy)-prop-1-inyl]-9(10)-estren-5α,17β-diol

in 100 ml Ethanol und 2 ml Triethylamin wird unter Zusatz von 200 mg Pd/$BaSO_4$ (10%) bei Raumtemperatur und Normaldruck hydriert. Nach Aufnahme von 80 ml Wasserstoff wird abgebrochen, vom Katalysator abfiltriert, eingeengt und der Rückstand an $Al_2O_3$ (III, neutral) mit n-Hexan/Essigester chromatographiert. Man erhält 1,50 g

3,3-(2,2-Dimethylpropan-1,3-dioxy)-11β-(4-dimethylaminophenyl)-17α-[3-(tetrahydropyran-2-yloxy)prop-1(Z)-enyl]-9(10)-estren-5α,17β-diol

als farblosen Schaum.

$^1$H–NMR ($CDCl_3$): δ = 5,3–5,8 ppm

H H
| |
(m, –C=C–, $J_{cis}$ = 12 Hz)

90 MHz

b) 1,1 g (1,7 mmol) des unter a) erhaltenen Produktes werden in 10 ml 70% Essigsäure über Nacht bei Raumtemperatur unter Argon gerührt. Anschliessend wird zur Vervollständigung der Reaktion 1 Stunde bei 50 °C gerührt. Zur Aufarbeitung giesst man auf ein Gemisch aus 25 g Eis/10 ml konzentriertem Ammoniakwasser und extrahiert mit Essigester. Nach dem Trocknen über $Na_2SO_4$ wird eingeengt und an Kieselgel mit n-Hexan/Essigester chromatographiert. Man erhält 0,42 g

11β-(4-Dimethylaminophenyl)-17β-hydroxy-17α-(3-hydroxyprop-1(Z)-enyl)-4,9(10)-estradien-3-on

als zitronengelben festen Schaum.

$[\alpha]_D$ ($CHCl_3$) = +203,5°

Beispiel 2

11β-(4-Dimethylaminophenyl)-17β-hydroxy-17α-(3-hydroxyprop-1(E)-enyl)-4,9(10)-estradien-3-on

a) Zu einer Lösung von 4,0 g (6,3 mmol)

3,3-(2,2-Dimethylpropan-1,3-dioxy)-11β-(4-dimethylaminophenyl)-17α-[3-(tetrahydropyran-2-yloxy)prop-1-inyl]-9(10)-estren-5α,17β-diol

in 100 ml absolutem Tetrahydrofuran werden unter Argon portionsweise 4,8 g (60,13 mol) Lithiumaluminiumhydrid gegeben. Danach wird das Reaktionsgemisch 2 Stunden bei Raumtemperatur und anschliessend 1 Stunde bei 50 °C gerührt. Unter Eiskühlung werden anschliessend nacheinander 4,8 ml Wasser, 4,8 ml 4 N NaOH und 14,4 ml Wasser zugetropft. Der gebildete Niederschlag wird mit einem Wasser/Essigester-Gemisch aufgeschlämmt, über eine Fritte abgesaugt und gründlich mit Essigester nachgewaschen. Die Essigester-Phase wird mit $Na_2SO_4$ getrocknet, eingeengt und mit n-Hexan/Essigester an $Al_2O_3$ (III, neutral) chromatographiert. Man erhält 1,05 g

3,3-(2,2-Dimethylpropan-1,3-dioxy)-11β-(4-dimethylaminophenyl)-17α-(3-hydroxyprop-1(E)-enyl)-9(10)-estren-5α,17β-diol

als farblosen Schaum.

$^1$H–NMR ($CDCl_3$): δ = 5,5–6,0 ppm

```
      H
      |
(m, –C=C–;
        |
        H
```

$J_{trans}$ = 15 Hz) 90 MHz

b) 1,0 g (1,8 mmol) des unter a) erhaltenen Produkts werden in 30 ml 70% Essigsäure über Nacht bei Raumtemperatur unter Argon gerührt. Man giesst auf ein Gemisch aus 25 g Eis/10 ml konzentriertem Ammoniakwasser und extrahiert mit Essigester. Nach dem Trocknen über $Na_2SO_4$ wird eingeengt und an Kieselgel mit n-Hexan/Essigester chromatographiert. Man erhält 0,59 g

11β-(4-Dimethylaminophenyl)-17β-hydroxy-17α-(3-hydroxyprop-1(E)-enyl)-4,9(10)-estradien-3-on

als zitronengelben festen Schaum.

$[\alpha]_D$ ($CHCl_3$) = +157,3°

Beispiel 3

17β-Hydroxy-17α-(3-hydroxyprop-1(Z)-enyl)-11β-(4-methylaminophenyl)-4,9(10)-estradien-3-on

a) Eine Lösung von 2,6 g des in Beispiel 1a) hergestellten Produkts in 100 ml Methylenchlorid wird unter Zusatz von 3,55 g Braunstein ($MnO_2$) 6 Stunden bei Raumtemperatur gerührt. Nach dem Absaugen wird mit Methylenchlorid nachgewaschen, eingeengt und mit n-Hexan/Essigester an $Al_2O_3$ (III, neutral) chromatographiert. Man erhält neben 1,3 g Ausgangsmaterial 0,86 g

3,3-(2,2-Dimethylpropan-1,3-dioxy)-11β-(4-methylaminophenyl)-17α-[3-(tetrahydropyran-2-yloxy)prop-1(Z)-enyl]-9(10)-estren-5α17β-diol

als farblosen festen Schaum.

b) 0,86 des unter a) erhaltenen Produkts werden in 8,6 ml 70% Essigsäure über Nacht bei Raumtemperatur unter Argon gerührt. Zur Vervollständigung der Reaktion wird danach 1,5 Stunden bei 50 °C gerührt. Das Gemisch wird anschliessend auf Wasser gegossen und mit konzentriertem Ammoniakwasser auf pH 10 gebracht. Es wird mit Methylenchlorid extrahiert, mit Wasser neutral gewaschen, über $Na_2SO_4$ getrocknet, eingeengt und der Rückstand mit n-Hexan/Essigester an $Al_2O_3$ (III, neutral) chromatographiert. Man erhält 0,25 g

17β-Hydroxy-17α-(3-hydroxyprop-1(Z)-enyl-11β-(4-methylaminophenyl)-4,9(10-estradien-3-on

als zitronengelben festen Schaum.

$^1$H–NMR ($CDCl_3$): δ = 6,98; 6,52 (dd, 4H, aromatische C–H, J = 8 Hz),
5,7–5,6 (m, 2H, –CH=CH–, $J_{cis}$ = 12,5 Hz),
5,74 (s, 1H, H-4),
4,4–4,2 (m, 2H, –$CO_2$CH)
2,80 (s, 3H, –$NCH_3$)
0,62 (s, 3H, $CH_3$)

Alternative Darstellung:

Darstellung von p-Brom-N-methyl-N-trimethylsilylanilin

Zu 20 g p-Brom-N-methylanilin in 50 ml absoluten Tetrahydrofuran wird unter Schutzgas eine Lithiumdiisopropylamidlösung, die aus 12,12 g Diisopropylamin in 100 ml absolutem Tetrahydrofuran und 68 ml 1,6 m n-Butyllithium/n-Hexan hergestellt wurde, bei einer Temperatur von −50 °C getropft, wobei sich ein farbloser Niederschlag bildet. Nachdem noch 1 Stunde bei −20 °C nachgerührt wurde, tropft man 11,6 g (= 13,5 ml) Trimethylsilylchlorid zu, wobei sich der Niederschlag wieder auflöst. Es wird 16 Stunden bei Raumtemperatur nachgerührt. Das bei der Reaktion gebildete Lithiumchlorid wird abgesaugt, das Filtrat eingeengt und der Rückstand im Wasserstrahlvakuum destilliert. Man erhält so 23,9 g

p-Brom-N-methyl-N-trimethylsilylanilin

vom Siedepunkt (16 Torr) 131–135 °C.

a) Zu einer Suspension von 2,25 g Magnesium in 20 ml absolutem Tetrahydrofuran und 0,1 ml Methyljodid tropft man unter Argon 23,9 g p-Brom-N-methyl-N-trimethylsilylanilin in 10 ml absolutem Tetrahydrofuran. Man beobachtet eine schwach exotherme Reaktion, und zur vollständigen Auflösung des Magnesiums wird noch 4 Stunden bei +50 °C gerührt. Dann kühlt man auf +5 °C ab und gibt 1 g Kupferchlorid zu. Nachdem 15 Minuten nachgerührt wurde, tropft man 10 g

3,3-(2,2-Dimethylpropan-1,3-dioxy)-5α,10α-epoxy-17α-[3-(tetrahydropyran-2-yloxy)prop-1-inyl]-9(11)-estren-17β-ol

in 50 ml absolutem Tetrahydrofuran zu. Man rührt noch 20 Stunden bei Raumtemperatur nach. Zur Aufarbeitung giesst man die Mischung in 100 g Eis/10 ml gesättigte Ammoniumchloridlösung und extrahiert mit Essigester. Die organischen Phasen werden mit Wasser neutral gewaschen und über Natriumsulfat getrocknet. Der eingeengte Rückstand wird an $Al_2O_3$ (III, neutral) mit n-Hexan/Essigester chromatographiert. Man erhält 10,7 g
3,3-(2,2-Dimethylpropan-1,3-dioxy)-11β-(4-methyl-aminophenyl)-17α-[3-(tetrahydropyran-2-yloxy)prop-1-inyl]-9(10)-estren-5α,17β-diol
als farblosen festen Schaum.

b) 10,7 g der unter a) gewonnenen Verbindung werden in 250 ml absolutem Ethanol und 9,6 ml Triethylamin in Gegenwart von 0,96 g Palladium/$BaSO_4$ (10%) unter Normalbedingungen hydriert. Nach der Aufnahme von 300 ml Wasserstoff wird abgebrochen, der Katalysator abfiltriert, eingeengt und der Rückstand an $Al_2O_3$ (III, neutral) mit n-Hexan/10–70% Essigester chromatographiert. Es werden 6,3 g
3,3-(2,2-Dimethylpropan-1,3-dioxy)-11β-(4-methylaminophenyl)-17α-[3-(tetrahydropyran-2-yloxy)prop-1(Z)-enyl]-9(10)-estren-5α,17β-diol
als farbloser fester Schaum erhalten.
$^1$H–NMR ($CDCl_3$): δ = 6,98; 6,52 ppm (dd, 4H, arom. C–H, 90 MHz
J = 8 Hz)
5,7–5,6 (m, 2H, –CH=CH–,
$J_{cis}$ = 12,5 Hz)

c) 4,25 g des unter b) erhaltenen Produkts löst man in 100 ml 70%iger Essigsäure und rührt 1 Stunde bei 50 °C. Dann rührt man die Mischung in 100 g Eis/100 ml konz. wässrige Ammoniaklösung ein und extrahiert mit Essigester. Die organischen Phasen werden mit Wasser neutral gewaschen und über Natriumsulfat getrocknet. Nach dem Einengen wird an Kieselgel mit einem n-Hexan/Essigestergemisch chromatographiert. Man erhält 1,86 g
17β-Hydroxy-17α-(3-hydroxy-prop-1(Z)-enyl)-11β-(4-methylaminophenyl)-4,9(10)-estradien-3-on
als zitronengelben festen Schaum.
$[\alpha]_D$ ($CHCl_3$) = +196,2°

Beispiel 4
11β-(4-Aminophenyl)-17β-hydroxy-17α-(3-hydroxyprop-1(Z)-enyl)-4,9(10)-estradien-3-on

a) Zu einer Suspension aus 2,64 g Magnesium und 0,05 ml Methyliodid in 20 ml absolutem Tetrahydrofuran gibt man unter Argon eine Lösung von 18,5 g N,N-Bis-[trimethyl-silyl]-4-bromanilin (J. Org. Chem. 40, 1090 (1975)) in 50 ml absolutem Tetrahydrofuran in der Weise, dass die Temperatur nach dem Anspringen der Reaktion 45 °C nicht übersteigt. Nach vollständiger Auflösung des Magnesiums kühlt man auf 0 °C, gibt 500 mg CuCl zu und rührt 15 Minuten bei 0 °C. Danach wird eine Lösung von 6,0 g
3,3-(2,2-Dimethylpropan-1,3-dioxy)-5α,10α-epoxy-17α-[3-(tetrahydropyran-2-yloxy)prop-1-inyl]-9(11)-estren-17β-ol
in 80 ml absolutem Tetrahydrofuran zugetropft und das Reaktionsgemisch 16 Stunden bei Raumtemperatur gerührt. Zur Aufarbeitung giesst man in wässrige Ammoniaklösung und extrahiert mit Essigester. Nach dem Trocknen der organischen Phase über $Na_2SO_4$ wird filtriert, eingeengt und der Rückstand 4 Stunden bei 50 °C unter Argon in 200 ml 20%iger ethanolischer Kaliumhydroxidlösung gerührt. Anschliessend wird in ein Eis/Wasser-Gemisch eingerührt und mit Essigester extrahiert. Die organische Phase wird mit Wasser neutral gewaschen und über $Na_2SO_4$ getrocknet. Nach dem Einengen wird an $Al_2O_3$ (III, neutral) mit Hexan/Essigester chromatographiert. Man erhält 5,27 g
11β-(4-Aminophenyl)-3,3-(2,2-dimethylpropan-1,3-dioxy)-17α-[3-(tetrahydropyran-2-yloxy)-prop-1-inyl]-9(10)-estren-5α,17β-diol
als farblosen festen Schaum.

b) 3,8 g des unter a) erhaltenen Produkts werden in 100 ml Ethanol und 3,6 ml Triethylamin unter Zusatz von 360 mg Palladium/$BaSO_4$ (10%) unter Normalbedingungen hydriert. Nach Aufnahme von 141 ml Wasserstoff wird abgebrochen, vom Katalysator abfiltriert, eingeengt und der Rückstand mit n-Hexan/Essigester an $Al_2O_3$ (III, neutral) chromatographiert. Man erhält 2,98 g
11β-(4-Aminophenyl)-3,3-(2,2-dimethylpropan-1,3-dioxy)-17α-[3-(tetrahydropyran-2-yloxy)prop-1(Z)-enyl]-9(10)-estren-5α,17β-diol
als farblosen festen Schaum.
$^1$H–NMR ($CDCl_3$): δ = 5,3–5,7 ppm

H H
| |
(m, –C=C–; $J_{cis}$ = 12,5 Hz)

90 MHz

c) 2,97 g des unter b) erhaltenen Produkts werden in 50 ml 70% Essigsäure über Nacht bei Raumtemperatur unter Argon gerührt. Zur Aufarbeitung wird auf 50 g Eis/50 ml konzentriertem Ammoniakwasser gegossen und mit Essigester extrahiert. Nach dem Trocknen über $Na_2SO_4$ wird eingeengt und der Rückstand an Kieselgel mit n-Hexan/Essigester chromatographiert. Man erhält 1,57 g
11β-(4-Aminophenyl)-17β-hydroxy-17α-(3-hydroxyprop-1(Z)-enyl)-4,9(10)-estradien-3-on
als zitronengelben festen Schaum.
$[\alpha]_D$ ($CHCl_3$) = +185,7°

Beispiel 5
11β-(4-Dimethylaminophenyl)-17β-hydroxy-17α-(3-hydroxyprop-1(Z)-enyl)-16β-methyl-4,9(10)-estradien-3-on

a) 2,67 g (4,1 mmol)
3,3-(2,2-Dimethylpropan-1,3-dioxy)-11β-(4-dimethylaminophenyl)-16β-methyl-17α-[3-(tetrahydropyran-2-yloxy)prop-1-inyl]-9(10)-estren-5α,17β-diol
werden in 100 ml Ethanol unter Zusatz von 2,3 ml Triethylamin und 235 mg Pd/$BaSO_4$ (10%) bei Raumtemperatur unter Normaldruck hydriert. Nach Aufnahme von 100 ml Wasserstoff wird vom

Katalysator abfiltriert, eingeengt und der Rückstand an $Al_2O_3$ (III, neutral) mit n-Hexan/Essigester chromatographiert. Man erhält 1,9 g
3,3-(2,2-Dimethylpropan-1,3-dioxy)-11β-(4-dimethylaminophenyl)-16β-methyl-17α-[3-(tetrahydropyran-2-yloxy)-prop-1(Z)-enyl]-9(10)-estren-5α,17β-diol
als farblosen festen Schaum.
$^1H$-NMR ($CDCl_3$): δ = 5,3–5,7 ppm

H H
| |
(m, –C=C–)

90 MHz

b) 1,9 g (2,9 mmol) des unter a) erhaltenen Produkts werden in 40 ml 70% Essigsäure über Nacht bei Raumtemperatur unter Argon gerührt. Zur Aufarbeitung wird auf 40 g Eis/40 ml konzentriertem Ammoniakwasser gegossen und mit Essigester extrahiert. Nach dem Trocknen über $Na_2SO_4$ wird eingeengt und der Rückstand an Kieselgel mit n-Hexan/Essigester chromatographiert. Man erhält 0,92 g
11β-(4-Dimethylaminophenyl)-17β-hydroxy-17α-(3-hydroxyprop-1(Z)-enyl)-16β-methyl-4,9(10)-estradien-3-on
als zitronengelben festen Schaum.
$[\alpha]_D$ ($CHCl_3$) = +210,8°

Beispiel 6

11β-(4-Dimethylaminophenyl)-17β-hydroxy-17α-(3-hydroxyprop-1(Z)-enyl)-16α-methyl-4,9(10)-estradien-3-on

a) 700 mg (1,08 mmol)
3,3-(2,2-Dimethylpropan-1,3-dioxy)-11β-(4-dimethylaminophenyl)-16α-methyl-17α-[3-(tetrahydropyran-2-yloxy)prop-1-inyl]-9(10)-estren-5α,17β-diol
werden in 100 ml Ethanol unter Zusatz von 0,62 ml Triethylamin und 62 mg Pd/$BaSO_4$ (10%) bei Raumtemperatur unter Normaldruck hydriert. Nach Aufnahme von 24 ml Wasserstoff wird vom Katalysator abfiltriert, eingeengt und der Rückstand von $Al_2O_3$ (III, neutral) mit n-Hexan/Essigester chromatographiert. Man erhält 525 mg
3,3-(2,2-Dimethylpropan-1,3-dioxy)-11β-(4-dimethylaminophenyl)-16α-methyl-17α-[3-(tetrahydropyran-2-yloxy)prop-1(Z)-enyl]-9(10)-estren-5α,17β-diol
als farblosen festen Schaum.
$^1H$-NMR ($CDCl_3$): δ = 5,4–5,8 ppm

H H
| |
(m, –C=C–, $J_{cis}$ = 12,5 Hz)

90 MHz

b) 500 mg (0,77 mmol) des unter a) erhaltenen Produkts werden in 20 ml 70% Essigsäure über Nacht bei Raumtemperatur unter Argon gerührt. Zur Aufarbeitung wird auf 20 g Eis/20 ml konzentriertem Ammoniakwasser gegossen und mit Essigester extrahiert. Nach dem Trocknen über $Na_2SO_4$ wird eingeengt und der Rückstand an Kieselgel mit n-Hexan/Essigester chromatographiert. Man erhält 289 mg
11β-(4-Dimethylaminophenyl)-17β-hydroxy-17α-(3-hydroxyprop-1(Z)-enyl)-16α-methyl-4,9(10)-estradien-3-on
als zitronengelben festen Schaum.
$[\alpha]_D$ ($CHCl_3$) = +194,6°

Beispiel 7

17β-Hydroxy-17α-(3-hydroxyprop-1(Z)-enyl)-11β-[4-(piperidin-1-yl)phenyl]-4,9(10)-estradien-3-on

a) 5,3 g (7,9 mmol)
3,3-(2,2-Dimethylpropan-1,3-dioxy)-11β-[4-(piperidin-1-yl)phenyl]-17α-[3-(tetrahydropyran-2-yloxy)prop-1-inyl]-9(10)-estren-5α,17β-diol
werden in 220 ml Ethanol unter Zusatz von 4,5 ml Triethylamin und 450 mg Pd/$BaSO_4$ (10%) bei Raumtemperatur unter Normaldruck hydriert. Nach Aufnahme von 180 ml Wasserstoff wird vom Katalysator abfiltriert, eingeengt und der Rückstand an $Al_2O_3$ (III, neutral) mit n-Hexan/Essigester chromatographiert. Man erhält 3,41 g
3,3-(2,2-Dimethylpropan-1,3-dioxy)-11β-[4-(piperidin-1-yl)phenyl]-17α-[3-(tetrahydropyran-2-yloxy)prop-1(Z)-enyl]-9(10)-estren-5α,17β-diol
als farblosen festen Schaum.
$^1H$-NMR ($CDCl_3$): δ = 5,3–5,8 ppm

H H
| |
(m, –C=C–; $J_{cis}$ = 12 Hz)

90 MHz

b) 3,37 g (5 mmol) des unter a) erhaltenen Produkts werden in 60 ml 70% Essigsäure über Nacht bei Raumtemperatur unter Argon gerührt. Anschliessend wird 1 Stunde bei 50 °C gerührt. Zur Aufarbeitung wird auf 60 g Eis/60 ml konzentriertem Ammoniakwasser gegossen und mit Essigester extrahiert. Nach dem Trocknen über $Na_2SO_4$ wird eingeengt und der Rückstand an Kieselgel mit n-Hexan/Essigester chromatographiert. Man erhält 1,75 g
17β-Hydroxy-17α-(3-hydroxyprop-1(Z)-enyl)-11β-[4-(piperidin-1-yl)phenyl]-4,9(10)-estradien-3-on
als zitronengelben festen Schaum.
$[\alpha]_D$ ($CHCl_3$) = +209°

Beispiel 8

17β-Hydroxy-17α-(3-hydroxyprop-1(Z)-enyl)-11β-[4-(pyrrolidin-1-yl)phenyl]-4,9(10)-estradien-3-on

a) 4,36 g (6,6 mmol)
3,3-(2,2-Dimethylpropan-1,3-dioxy)-11β-[4-(pyrrolidin-1-yl)phenyl]-17α-[3-(tetrahydropyran-2-yloxy)prop-1-inyl]-9(10)-estren-5α,17β-diol
werden in 200 ml Ethanol unter Zusatz von 3,8 ml Triethylamin und 380 mg Pd/$BaSO_4$ (10%) bei Raumtemperatur unter Normaldruck hydriert. Nach Aufnahme von 160 ml Wasserstoff wird vom Katalysator abfiltriert, eingeengt und der Rückstand an $Al_2O_3$ (III, neutral) mit n-Hexan/Essigester

chromatographiert. Man erhält 2,91 g
3,3-(2,2-Dimethylpropan-1,3-dioxy)-11β-[4-(pyrrolidin-1-yl)phenyl]-17α-[3-(tetrahydropyran-2-yloxy)prop-1(Z)-enyl]-9(10)-estren-5α,17β-diol
als farblosen festen Schaum.
$^1$H–NMR ($CDCl_3$): δ = 5,3–5,8 ppm

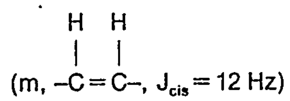

90 MHz

b) 2,91 g (4,4 mmol) des unter a) erhaltenen Produkts werden in 50 ml 70% Essigsäure 5 Stunden bei 50 °C gerührt. Zur Aufarbeitung wird auf 50 g Eis/50 ml konzentriertem Ammoniakwasser gegossen und mit Essigester extrahiert. Nach dem Trocknen über $Na_2SO_4$ wird eingeengt und der Rückstand an Kieselgel mit n-Hexan/Essigester chromatographiert. Man erhält 1,66 g
17β-Hydroxy-17α-(3-hydroxyprop-1(Z)-enyl)-11β-[4-(pyrrolidin-1-yl)phenyl]-4,9(10)-estradien-3-on
als zitronengelben festen Schaum.
$[\alpha]_D$ ($CHCl_3$) = +214,1°

Beispiel 9

11β-[4-(2,5-Dimethylpyrrol-1-yl)phenyl]-17β-hydroxy-17α-(3-hydroxyprop-1(Z)-enyl)-4,9(10)-estradien-3-on

a) 1,4 g (2,1 mmol)
3,3-(2,2-Dimethylpropan-1,3-dioxy)-11β-[4-(2,5-dimethylpyrrol-1-yl)phenyl]-17α-[3-(tetrahydropyran-2-yloxy)prop-1-inyl]-9(10)-estren-5α,17β-diol
werden in 60 ml Ethanol unter Zusatz von 1,2 ml Triethylamin und 120 mg Pd/$BaSO_4$ (10%) bei Raumtemperatur und Normaldruck hydriert. Nach Aufnahme von 48 ml Wasserstoff wird vom Katalysator abfiltriert, eingeengt und der Rückstand an $Al_2O_3$ (III, neutral) mit n-Hexan/Essigester chromatographiert. Man erhält 758 mg
3,3-(2,2-Dimethylpropan-1,3-dioxy)-11β-[4-(2,5-dimethylpyrrol-1-yl)phenyl]-17α-[3-(tetrahydropyran-2-yloxy)prop-1(Z)-enyl]-9(10)-estren-5α,17β-diol
als farblosen festen Schaum.
$^1$H–NMR ($CDCl_3$): δ = 5,4–5,8 ppm

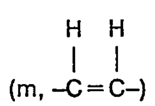

90 MHz

b) 2,57 g (3,7 mmol) des nach a) gewonnenen Produkts werden in 50 ml Ethanol unter Zusatz von 300 mg p-Toluolsulfonsäure-Monohydrat 2 Stunden bei Raumtemperatur unter Argon gerührt. Zur Aufarbeitung wird auf 50 ml Eiswasser/5 ml konzentrierter Ammoniaklösung gegossen und mit Essigester extrahiert. Nach dem Trocknen über $Na_2SO_4$ wird eingeengt und der Rückstand an Kieselgel mit n-Hexan/Essigester chromatographiert. Man erhält 0,85 g
11β-[4-(2,5-Dimethylpyrrol-1-yl)phenyl]-17β-hydroxy-17α-(3-hydroxyprop-1(Z)-enyl)-4,9(10)-estradien-3-on
als gelben festen Schaum.
$[\alpha]_D$ ($CHCl_3$) = +152,4°

Beispiel 10

11β-(4-Dimethylaminophenyl)-16(E)-ethyliden-17β-hydroxy-17α-(3-hydroxyprop-1(Z)-enyl)-4,9(10)-estradien-3-on

a) Eine Suspension aus 29,3 g
3,3-(2,2-Dimethyl-propan-1,3-dioxy)-5(10),9(11)-estradien-17-on
und 28,6 g Bis-dimethylamino-tert.-butoxymethan wird unter Argon 60 Minuten bei 160 °C gerührt. Nach dem Abkühlen verreibt man das Rohprodukt mit ca. 50 ml Ethylacetat, filtriert und kristallisiert den Filtrierrückstand aus Ethylacetat um. Auf diese Weise erhält man 27,6 g
16-Dimethylaminomethylen-3,3-(2,2-dimethylpropan-1,3-dioxy)-5(10),9(11)-estradien-17-on
vom Schmelzpunkt 208–211 °C.

b) Eine Lösung von 14,4 g
16-Dimethylaminomethylen-3,3-(2,2-dimethylpropan-1,3-dioxy)-5(10),9(11)-estradien-17-on
in 220 ml Toluol wird unter Eiswasserkühlung tropfenweise mit 85 ml einer 5%igen Lösung von Methyllithium in Diethylether versetzt. Nach Zugabe rührt man 15 Minuten bei +5 bis +10 °C, zersetzt überschüssiges Reagenz durch vorsichtige Zugabe von ca. 20 ml Wasser, giesst die Reaktionslösung anschliessend in ca. 3 l Eiswasser und extrahiert mit Methylenchlorid. Das Rohprodukt wird an neutralem Aluminiumoxid mit Hexan/Ethylacetat chromatographiert. Nach Kristallisation der Hauptfraktion aus Ethylacetat erhält man 13,0 g
3,3-(2,2-Dimethyl-propan-1,3-dioxy)-16(E)-ethyliden-5(10),9(11)-estradien-17-on
vom Schmelzpunkt 121–123 °C.

c) Zu einer Lösung von 9,4 g
3,3-(2,2-Dimethyl-propan-1,3-dioxy)-16(E)-ethyliden-5(10),9(11)-estradien-17-on
in 43 ml Methylenchlorid, 0,34 ml Hexachloraceton und 0,01 ml Pyridin tropft man unter Eiswasserkühlung 4,3 ml 30%iges Wasserstoffperoxid und rührt anschliessend 16 Stunden bei 25 °C. Zur Aufarbeitung verdünnt man die Reaktionslösung mit ca. 100 ml Methylenchlorid, wäscht nacheinander mit 5%iger $Na_2S_2O_3$-Lösung und Wasser, trocknet die Methylenchlorid-Phase über $Na_2SO_4$ und engt ein. Das so erhaltene 5,10-Epoxidgemisch wird an $Al_2O_3$ (neutral, Stufe III) mit Hexan/Ethylacetat chromatographiert. Man erhält 4,7 g
3,3-(2,2-Dimethyl-propan-1,3-dioxy)-5α,10α-epoxy-16(E)-ethyliden-9(11)-estren-17-on
vom Schmelzpunkt 139–141 °C (Ethylacetat/Diisopropylether).

d) Eine Lösung von 1,95 g 3-(Tetrahydropyran-2-yloxy)-1-propin in 41 ml absoluten Tetrahydrofuran (THF) wird bei 0 bis +5 °C tropfenweise mit 11,3 ml einer 15%igen Lösung von n-Butyllithium in Hexan versetzt. Anschliessend tropft man eine Lösung von 2,25 g

3,3-(2,2-Dimethyl-propan-1,3-dioxy)-5α,10α-epoxy-16(E)-ethyliden-9(11)-estren-17-on
hinzu und rührt 60 Minuten bei Raumtemperatur nach. Zur Aufarbeitung giesst man in Eiswasser und extrahiert mit Ethylacetat. Das Rohprodukt wird aus Diisopropylether kristallisiert. Man erhält 2,8 g
3,3-(2,2-Dimethyl-propan-1,3-dioxy)-5α,10α-epoxy-16(E)-ethyliden-17α-[3-(tetrahydropyran-2-yloxy)-1-propinyl]-9(11)-estren-17β-ol
vom Schmelzpunkt 196–198 °C.

e) Zu einer Suspension von 604 mg Magnesium in 10 ml absoluten THF gibt man bei Raumtemperatur sukzessive 0,05 ml Methyliodid und tropfenweise eine Lösung von 6,42 g 4-Brom-dimethylanilin in 30 ml absoluten THF so hinzu, dass die Innentemperatur 45 °C nicht übersteigt. Nach vollständiger Auflösung des Magnesiums kühlt man auf 0 °C, gibt 124 mg CuCl (fest) hinzu und rührt 15 Minuten bei 0 °C. Danach wird eine Lösung von 3,5 g
3,3-(2,2-Dimethyl-propan-1,3-dioxy)-5α,10α-epoxy-16(E)-ethyliden-17α-[3-(tetrahydropyran-2-yloxy)-1-propinyl]-9(11)-estren-17β-ol
in 25 ml absoluten THF hinzugetropft und anschliessend 20 Stunden bei 25 °C gerührt. Zur Aufarbeitung giesst man in wässrige $NH_3$-Lösung und extrahiert mit Ethylacetat. Das Rohprodukt wird an $Al_2O_3$ mit Hexan/Ethylacetat chromatographiert. Die ölige Hauptfraktion (4,3 g) wird in die Folgestufe eingesetzt.

f) Eine Lösung von 4,3 g des unter e) erhaltenen
11β-(4-Dimethylaminophenyl)-3,3-(2,2-dimethylpropan-1,3-dioxy)-16(E)-ethyliden-17α-[3-(tetrahydropyran-2-yloxy)-1-propinyl]-9(10)-estren-5α,17β-diol
in 300 ml Ethanol und 7 ml Triethylamin wird nach Zusatz von 500 mg Pd/$BaSO_4$ (10%) bei Raumtemperatur und Normaldruck hydriert. Nach Aufnahme von 140 ml Wasserstoff wird abgebrochen, vom Katalysator abfiltriert und eingeengt. Man erhält 4,3 g
11β-(4-Dimethylaminophenyl)-3,3-(2,2-dimethylpropan-1,3-dioxy)-16(E)-ethyliden-17α-[3-(tetrahydropyran-2-yloxy)-prop-1(Z)-enyl]-9(10)-estren-5α,17β-diol
als farbloses Öl.

g) Eine Lösung von 3,63 g des unter f) erhaltenen Produkts in 17 ml 70%iger wässriger Essigsäure wird 20 Stunden bei Raumtemperatur unter Argon gerührt. Danach giesst man in Eiswasser, stellt durch Zugabe von konzentrierter wässriger $NH_3$-Lösung einen pH-Wert von ca. 10,5 ein und extrahiert mit Ethylacetat. Das Rohprodukt wird an Kieselgel mit Hexan/Ethylacetat chromatographiert. Die Hauptfraktion wird aus Hexan/Ethanol kristallisiert. Man erhält 1,3 g
11β-(4-Dimethylaminophenyl)-16(E)-ethyliden-17β-hydroxy-17α-(3-hydroxyprop-1(Z)-enyl)-4,9(10)-estradien-3-on
vom Schmelzpunkt 127–130 °C (die Verbindung kristallisiert mit 1 Mol Ethanol).

Beispiel 11
11β-(Dimethylaminophenyl)-17β-hydroxy-17α-(3-hydroxyprop-1(Z)-enyl)-4,9(10)-estradien-3-on

a) 11β-(4-Dimethylaminophenyl)-3,3-ethylendioxy-17α-(3-hydroxyprop-1(Z)-enyl)-9-estren-5α,17β-diol
Eine Lösung von 500 mg
11β-(4-Dimethylaminophenyl)-3,3-ethylendioxy-17α-(3-hydroxy-1-propinyl)-9-estren-5α,17β-diol
in 5 ml Ethanol wird nach Zusatz von 5 mg Lindlar-Katalysator (Pd/$CaCO_3$ (5%) unter Zusatz von Pb (0 Acetyl)$_2$) 16 Stunden bei Raumtemperatur und unter Normaldruck hydriert. Die Wasserstoffaufnahme betrug 22,7 ml. Man saugt die Reaktionslösung über Celite ab, wäscht mit Essigester nach und engt im Vakuum zur Trockne ein. Das Rohprodukt wird an 100 g Kieselgel mit einem Methylenchlorid-Aceton-Gradienten (0–20% Aceton) gereinigt. Man isoliert 420 mg
11β-(4-Dimethylaminophenyl)-3,3-ethylendioxy-17α-(3-hydroxyprop-1(Z)-enyl)-9-estren-5α,17β-diol
als farblosen Schaum.
$[\alpha]^{25}_D = -13°$.

b) 11β-(4-Dimethylaminophenyl)-17β-hydroxy-17α-(3-hydroxyprop-1(Z)-enyl)-4,9(10)-estradien-3-on
Eine Lösung von 255 mg
11β-(4-Dimethylaminophenyl)-3,3-ethylendioxy-17α-(3-hydroxyprop-1(Z)-enyl)-9-estren-5α,17β-diol
in 4 ml Ethanol wird mit 15 mg Pyridiniumtosylat 15 Minuten bei 55 °C Badtemperatur gerührt. Nach Zusatz weniger Tropfen Pyridin wird das Lösungsmittel im Vakuum abgedampft. Der Rückstand wird an 50 g Kieselgel mit n-Hexan/Essigester chromatographiert. Man isoliert 210 mg
11β-(4-Dimethylaminophenyl)-17β-hydroxy-17α-(3-hydroxyprop-1(Z)-enyl)-4,9(10)-estradien-3-on
als festen Schaum.

Beispiel 12
17α-(3-Acetoxyprop-1(Z)-enyl)-11β-(4-dimethylaminophenyl)-17β-hydroxy-4,9(10)-estradien-3-on

1,1 g
11β-(4-Dimethylaminophenyl)-17β-hydroxy-17α-(3-hydroxyprop-1(Z)-enyl)-4,9(10)-estradien-3-on
werden 3 Stunden in 20 ml Pyridin und 10 ml Acetanhydrid bei Raumtemperatur unter Schutzgas gerührt. Zur Aufarbeitung wird das Reaktionsgemisch in ein Eis/Wasser-Gemisch eingerührt und mit Essigester extrahiert. Die organische Phase wird nacheinander mit $NaHCO_3$-Lösung und Wasser gewaschen und über $Na_2SO_4$ getrocknet. Nach dem Einengen wird mit n-Hexan/Essigester an Kieselgel chromatographiert. Man erhält 1,15 g
17α-(3-Acetoxyprop-1(Z)-enyl)-11β-(4-dimethylaminophenyl)-17β-hydroxy-4,9(10)-estradien-3-on

als zitronengelben festen Schaum.
$[\alpha]_D$ ($CHCl_3$) = +197°.

Beispiel 13
11β-(4-N-Formyl-N-methylaminophenyl)-17β-hydroxy-17α-(3-hydroxyprop-1(Z)-enyl)-4,9(10)-estradien-3-on

3,15 g des unter 3a) erhaltenen Produkts löst man unter Argon in 60 ml absolutem Tetrahydrofuran und tropft bei einer Temperatur von −20 °C 0,9 ml Essigsäure-Ameisensäure-Anhydrid unter Feuchtigkeitsausschluss zu. Nach 1 Stunde Reaktionszeit bei Raumtemperatur rührt man die Lösung in 60 g Eis/5 ml konz. wässrige Ammoniaklösung ein und extrahiert sie mit Essigester. Die organischen Phasen werden mit Wasser neutral gewaschen, über Natriumsulfat getrocknet und eingeengt. Der erhaltene Rückstand wird mit 100 ml 70%iger Essigsäure 3 Stunden bei 50 °C gerührt. Die Lösung wird anschliessend auf 100 g Eis/100 ml konz. wässrige Ammoniaklösung gegossen und mit Methylenchlorid extrahiert. Die organischen Phasen werden mit Wasser neutral gewaschen und über Natriumsulfat getrocknet, eingeengt, und der Rückstand wird an Kieselgel mit Methylenchlorid/Aceton chromatographiert. Man erhält 1,84 g
11β-(4-N-Formyl-N-methylaminophenyl)-17β-hydroxy-17α-(3-hydroxyprop-1(Z)-enyl)-4,9(10)-estradien-3-on.
$[\alpha]_D$ ($CH_3OH$) = +185,7°

Beispiel 14
11β-(4-N-Acetyl-N-methylaminophenyl)-17β-hydroxy-17α-(3-hydroxyprop-1(Z)-enyl)-4,9(10)-estradien-3-on

3,15 g des unter 3a) erhaltenen Produkts werden in 40 ml absolutem Pyridin unter Argon gelöst. Bei +5 °C lässt man 0,41 ml Acetylchlorid in der Weise zutropfen, dass die Temperatur +10 °C nicht übersteigt. Es wird noch 1 Stunde bei +5 °C nachgerührt, dann lässt man die Lösung in 80 g Eis/40 ml konz. Salzsäure einfliessen und extrahiert mit Methylenchlorid. Die über Natriumsulfat getrockneten organischen Phasen werden eingeengt, und der erhaltene Rückstand wird in 100 ml 70%iger Essigsäure 3 Stunden bei 50 °C gerührt. Zur Aufarbeitung wird auf 100 g Eis/100 ml konz. wässrige Ammoniaklösung gegossen und mit Methylenchlorid extrahiert. Die organischen Phasen werden mit Wasser neutral gewaschen und über Natriumsulfat getrocknet. Nach dem Einengen wird der Rückstand an Kieselgel mit Methylenchlorid/20–50% Aceton chromatographiert. Man gewinnt 1,8 g
11β-(4-N-Acetyl-N-methylaminophenyl)-17β-hydroxy-17α-(3-hydroxyprop-1(Z)-enyl-4,9(10)-estradien-3-on.
$[\alpha]_D$ ($CH_3OH$) = +156,4°

Beispiel 15
11β-(4-Formylaminophenyl)-17β-hydroxy-17α-(3-hydroxyprop-1(Z)-enyl)-4,9(10)-estradien-3-on

1,2 g des unter 4b) erhaltenen Produkts werden in 40 ml absolutem Tetrahydrofuran unter Argon gelöst und bei einer Temperatur von −20 °C unter Feuchtigkeitsausschluss mit 0,52 ml Essigsäure-Ameisensäure-Anhydrid versetzt. Nach 30 Minuten Reaktionszeit wird die Lösung zur Aufarbeitung in 30 g Eis/5 ml konz. wässrige Ammoniaklösung eingerührt und mit Essigester extrahiert. Nach dem Trocknen über Natriumsulfat wird das erhaltene Rohprodukt zur Verseifung in 50 ml 70%ige Essigsäure gelöst und 3 Stunden bei 50 °C gerührt. Zur Aufarbeitung wird auf 50 g Eis/50 ml konz. wässrige Ammoniaklösung gegossen und mit Methylenchlorid extrahiert. Nach dem Trocknen über Natriumsulfat wird eingeengt und der Rückstand an Kieselgel mit Methylenchlorid/30–100% Aceton chromatographiert. Man erhält 0,62 g
11β-(4-Formylaminophenyl)-17β-hydroxy-17α-(3-hydroxyprop-1(Z)-enyl)-4,9(10)-estradien-3-on.
$[\alpha]_D$ ($CH_3OH$) = +193,7°

Beispiel 16
11β-(4-Acetylaminophenyl)-17β-hydroxy-17α-(3-hydroxyprop-1(Z)-enyl)-4,9(10)-estradien-3-on

1,6 g des unter 4b) erhaltenen Produkts werden in 40 ml absolutem Pyridin unter Argon gelöst. Bei +5 °C lässt man 0,21 ml Acetylchlorid in der Weise zutropfen, dass die Temperatur nicht über +10 °C steigt. Nachdem 4 Stunden bei Raumtemperatur nachgerührt wurde, wird die Lösung in 50 g Eis/40 ml konz. Salzsäure eingerührt und mit Methylenchlorid extrahiert. Nach dem Trocknen über Natriumsulfat wird das erhaltene Rohprodukt mit 70 ml 70%iger Essigsäure bei 50 °C 3 Stunden gerührt. Zur Aufarbeitung wird die Lösung in 70 g Eis/70 ml konz. wässrige Ammoniaklösung gegossen und mit Methylenchlorid extrahiert. Die organische Phase wird über Natriumsulfat getrocknet, eingeengt und der erhaltene Rückstand an Kieselgel mit Methylenchlorid/50–100% Aceton chromatographiert. Man erhält 0,97 g
11β-(4-Acetylaminophenyl)-17β-hydroxy-17α-(3-hydroxyprop-1(Z)-enyl)-4,9(10)-estradien-3-on.
$[\alpha]_D$ ($CH_3OH$) = +196°

Beispiel 17
11β-(4-Dimethylaminophenyl-17aβ-hydroxy-17aα-(3-hydroxyprop-1(Z)-enyl)-D-homo-4,9,16-estratrien-3-on

a) Eine Lösung von 22,4 g D-Homo-4,9,16-estratrien-3,17a-dion in 225 ml Methylenchlorid wird sukzessive mit 22,4 g 2,2-Dimethylpropan-1,3-diol, 11,2 ml Orthoameisensäuretrimethylester und 20 mg p-Toluolsulfonsäure versetzt. Man rührt 2,5 Stunden bei Raumtemperatur, verdünnt mit 150 ml Methylenchlorid, wäscht mit gesättigter Natriumhydrogencarbonatlösung, trocknet über Natriumsulfat und engt ein. Der ölige Rückstand wird über $Al_2O_3$ (neutral, III) mit Hexan/Essigester chromatographiert. Nach Kristallisation der Hauptfraktion aus Diisopropylether erhält man 19,1 g
3,3-(2,2-Dimethylpropan-1,3-dioxy)-D-homo-5(10),9(11),16-estratrien-17a-on
vom Schmelzpunkt 154–156 °C.

b) Zu einer Lösung von 19,1 g
3,3-(2,2-Dimethylpropan-1,3-dioxy)-D-homo-5(10),9(11)-16-estratrien-17a-on
in 75 ml Methylenchlorid, 0,6 ml Hexachloraceton und 0,1 ml Pyridin tropft man unter Eiswasserkühlung 7,6 ml 30%iges $H_2O_2$. Man rührt 16 Stunden bei Raumtemperatur, verdünnt mit 100 ml Methylenchlorid, wäscht mit 5%iger $Na_2S_2O_3$-Lösung und engt ein. Das so erhaltene Gemisch aus 5α,10α-Epoxid und 5β,10β-Epoxid wird durch Chromatographie an $Al_2O_3$ mit Hexan/Essigester getrennt. Man erhält nach Kristallisation aus Essigester/Diisopropylether 9,7 g
3,3-(2,2-Dimethylpropan-1,3-dioxy)-5α,10α-epoxy-D-homo-9(11),16-estradien-17a-on
vom Schmelzpunkt 188–191 °C.

c) Zu einer Lösung von 9,24 g
3-(Tetrahydropyran-2-yloxy)-1-propin
in 183 ml absolutem Tetrahydrofuran tropft man bei 0 °C unter Schutzgas 53 ml n-Butyllithium/n-Hexan (1,6 mol/l) und lässt 15 Minuten nachrühren. Dann wird eine Lösung von 9,1 g
3,3-(2,2-Dimethylpropan-1,3-dioxy)-5α,10α-epoxy-D-homo-9(11),16-estradien-17a-on
in 183 ml absolutem Tetrahydrofuran zugetropft und das Reaktionsgemisch 3 Stunden bei 0 °C nachgerührt. Zur Aufarbeitung wird vorsichtig mit Eis/Wasser zersetzt und mit Essigester extrahiert. Die organischen Phasen werden mit Wasser gewaschen, über Natriumsulfat getrocknet, mit Aktivkohle versetzt, über Celite abgesaugt und eingeengt. Man erhält so 17,4 g
3,3-(2,2-Dimethylpropan-1,3-dioxy)-5α,10α-epoxy-17aβ-hydroxy-17aα-[3-(tetrahydropyran-2-yloxy)prop-1-inyl]-D-homo-9(11),16-estradien
als gelbliches Öl, das ohne weitere Reinigung in die nächste Stufe eingesetzt wird.

d) Zu einer Suspension aus 2,27 g Magnesium und 0,05 ml Methyliodid in 42 ml absolutem Tetrahydrofuran wird eine Lösung von 24,8 g 4-Brom-N,N-dimethylanilin in 124 ml absolutem Tetrahydrofuran in der Weise getropft, dass die Temperatur nach dem Anspringen der Reaktion 45 °C nicht übersteigt. Zur vollständigen Auflösung des Magnesiums wird noch 1 Stunde nachgerührt, dann wird das Reaktionsgemisch auf 0 °C abgekühlt, mit 480 mg Kupfer(I)chlorid versetzt und 15 Minuten nachgerührt. Anschliessend wird eine Lösung von 17,4 g des unter c) erhaltenen Rohprodukts in 83 ml absolutem Tetrahydrofuran zugetropft, und es wird weitere 5 Stunden bei Raumtemperatur nachgerührt. Zur Aufarbeitung wird das Reaktionsgemisch in Eiswasser gegossen, mit Essigester versetzt, über Celite filtriert und das Filtrat mit Essigester extrahiert. Die organischen Phasen werden mit Wasser neutral gewaschen, über Natriumsulfat getrocknet, filtriert und eingeengt. Der Rückstand wird an $Al_2O_3$ (neutral, III) mit Hexan/Essigester chromatographiert. Man erhält so 10,8 g
3,3-(2,2-Dimethylpropan-1,3-dioxy)-11β-(4-dimethylaminophenyl)-17aα-[3-(tetrahydropyran-2-yloxy)prop-1-inyl]-D-homo-9(10),16-estradien-5α,17aβ-diol
als hellgelbes Öl.

e) 10,8 g des unter d) erhaltenen Produkts werden in 182 ml Tetrahydrofuran und 5,3 ml Pyridin unter Zusatz von 1,1 g Palladium/$BaSO_4$ (10%) unter Normalbedingungen hydriert. Nach Aufnahme von 410 ml Wasserstoff wird abgebrochen, das Hydriergemisch über Celite abgesaugt und eingeengt. Das so erhaltene Rohprodukt wird anschliessend in 55 ml 70%iger Essigsäure aufgenommen und 1 Stunde bei 60 °C unter Schutzgas gerührt. Zur Aufarbeitung wird nach dem Abkühlen auf Eiswasser gegossen, vorsichtig mit konz. wässriger Ammoniaklösung ein pH von 10–11 eingestellt und mit Essigester extrahiert. Die Essigester-Phasen werden mit Wasser neutral gewaschen, über Natriumsulfat getrocknet, filtriert und eingeengt. Aus dem Rückstand erhält man durch Chromatographie an Kieselgel mit Hexan/Essigester 5,1 g
11β-(4-Dimethylaminophenyl)-17aβ-hydroxy-17aα-(3-hydroxyprop-1(Z)-enyl)-D-homo-4,9,16-estratrien-3-on
als zitronengelben festen Schaum.

UV ($CH_3OH$): λ(ε) = 205 nm (36 800), 259 nm (17 400), 304 nm (21 500).

Beispiel 18

17β-Hydroxy-17α-(3-hydroxyprop-1(Z)-enyl)-11β-(4-methoxyphenyl)-4,9(10)-estra-dien-3-on

a) Zu einer Suspension von 970 mg Magnesium und 0,01 ml Methyliodid in 10 ml absolutem Tetrahydrofuran tropft man unter Argon eine Lösung von 7,46 g (5 ml) p-Bromanisol in 5 ml absolutem Tetrahydrofuran in der Weise, dass nach dem Anspringen der Reaktion eine Temperatur von 45 °C nicht überschritten wird. Nach vollständiger Auflösung des Magnesiums wird auf 0 °C gekühlt, und es werden 400 mg Kupfer-(I)-chlorid hinzugefügt. Man rührt noch 15 Minuten bei 0 °C und tropft dann eine Lösung von 3,9 g
3,3-(2,2-Dimethylpropan-1,3-dioxy-5α,10α-epoxy-17α-[3-(tetrahydropyran-2-yloxy)prop-1-inyl]-9(10)-estren-17β-ol
in 20 ml absolutem Tetrahydrofuran zu. Nach beendeter Zugabe wird über Nacht bei Raumtemperatur gerührt. Zur Aufarbeitung giesst man das Reaktionsgemisch in 40 g Eis/10 ml konz. Ammoniumchloridlösung und extrahiert mit Essigester. Die organischen Phasen werden mit Wasser gewaschen und über Natriumsulfat getrocknet. Anschliessend wird filtriert, eingeengt und der Rückstand an $Al_2O_3$ (III, neutral) mit n-Hexan/Essigester chromatographiert. Man erhält 4,1 g
3,3-(2,2-Dimethylpropan-1,3-dioxy)-11β-(4-methoxyphenyl)17α-[3-(tetrahydropyran-2-yloxy)-prop-1-inyl]-9(10)-estren-5α,17β-diol
als farblosen festen Schaum.

b) 4,1 g des unter a) erhaltenen Produkts werden in 50 ml Ethanol und 4 ml Pyridin in Gegenwart von 400 mg Palladium/$BaSO_4$ (10%) unter Normalbedingungen hydriert. Nach Aufnahme von 150 ml Wasserstoff wird abgebrochen, vom Katalysator abfiltriert und der Rückstand an $Al_2O_3$ (III,

neutral) mit n-Hexan/Essigester chromatographiert. Es werden 3,6 g
3,3-(2,2-Dimethylpropan-1,3-dioxy)-11β-(4-methoxyphenyl)-17α-[3-(tetrahydropyran-2-yloxy)prop-1(Z)-enyl]-9(10)-estren-5α,17β-diol
als farbloser fester Schaum erhalten.

$^1$H-NMR ($CDCl_3$): δ = 7,05; 6,75 ppm (dd, 4H, arom. CH,
J = 8 Hz)
90 MHz 5,8–5,5 (m, 2H, –CH=CH–,
$J_{cis}$ = 12 Hz)
3,75 (s, 3H, $-OCH_3$)

c) 3,5 g des unter b) erhaltenen Produkts werden in 50 ml 70%iger Essigsäure 2 Stunden bei 50 °C unter Schutzgas gerührt. Zur Aufarbeitung wird in 50 g Eis/50 ml konz. wässriger Ammoniaklösung eingerührt und mit Methylenchlorid extrahiert. Die organischen Phasen werden mit Wasser neutral gewaschen, über Natriumsulfat getrocknet, filtriert und eingeengt. Durch Chromatographie des Rückstandes an Kieselgel mit n-Hexan/Essigester erhält man 2,2 g
17β-Hydroxy-17α-(3-hydroxyprop-1(Z)-enyl)-11β-(4-methoxyphenyl)-4,9(10)-estradien-3-on
als hellgelben festen Schaum.

$^1$H-NMR ($CDCl_3$): δ = 7,03; 6,75 ppm
(dd, 4H, arom. CH, J = 8 Hz)
5,8–5,6 (m, 2H, –CH=CH–,
$J_{cis}$ = 12,5 Hz)
3,77 (s, 3H, $-OCH_3$)
$[\alpha]_D$ ($CHCl_3$) = +178°

Beispiel 19

17β-Hydroxy-17α-(3-hydroxyprop-1(Z)-enyl)-11β-(4-methylmercaptophenyl)-4,9(10)-estradien-3-on

a) Zu einer Suspension von 486 mg Magnesium und 0,01 ml Methyliodid in 10 ml absolutem Tetrahydrofuran tropft man unter Argon 4,06 g 4-Bromthioanisol in 10 ml absolutem Tetrahydrofuran in der Weise, dass die Temperatur nach dem Anspringen der Reaktion 45 °C nicht übersteigt. Nach der vollständigen Auflösung des Magnesiums wird das Reaktionsgemisch auf 0 °C gekühlt, und es werden 100 mg Kupfer(I)-chlorid hinzugefügt. Nachdem noch 15 Minuten nachgerührt wurde, tropft man eine Lösung von 3,68 g
3,3-(2,2-Dimethylpropan-1,3-dioxy)-5α,10α-epoxy-17α-[3-(tetrahydropyran-2-yloxy)prop-1-inyl]-9(11)-estren-17β-ol
in 20 ml absolutem Tetrahydrofuran zu und rührt über Nacht bei Raumtemperatur. Zur Aufarbeitung wird auf 40 g Eis/40 ml konz. Ammoniumchloridlösung gegossen und mit Essigester extrahiert. Die organischen Phasen werden mit Wasser gewaschen, über Natriumsulfat getrocknet, filtriert, eingeengt, und der Rückstand wird an $Al_2O_3$ (III, neutral) mit n-Hexan/Essigester chromatographiert. Man erhält so 3,41 g
3,3-(2,2-Dimethylpropan-1,3-dioxy)-11β-(4-methylmercaptophenyl)-17α-[3-(tetrahydropyran-2-yloxy)prop-1-inyl]-9(10)-estren-5α,17β-diol als farblosen festen Schaum.

b) 3,41 g des unter a) erhaltenen Produkts werden in 50 ml Ethanol und 4 ml Pyridin in Gegenwart von 350 mg Palladium/$BaSO_4$ (10%) unter Normalbedingungen hydriert. Nach Aufnahme von 125 ml Wasserstoff wird abgebrochen, vom Katalysator abfiltriert und der Rückstand an $Al_2O_3$ (III, neutral) mit n-Hexan/Essigester chromatographiert. Man erhält 3,04 g
3,3-(2,2-Dimethylpropan-1,3-dioxy)-11β-(4-methylmercaptophenyl)-17α-[3-(tetrahydropyran-2-yloxy)-prop-1(Z)-enyl]-9(10)-estren-5α,17β-diol
als farblosen festen Schaum.

$^1$H-NMR ($CDCl_3$): δ = 7,05 ppm (s, 4H, arom. CH)
90 MHz 5,75–5,5 (m, 2H, –CH=CH–,
$J_{cis}$ = 12 Hz)
2,36 (s, 3H, $-SCH_3$)

c) 2,9 g des unter b) erhaltenen Produkts werden in 40 ml 70%iger Essigsäure 2 Stunden bei 50 °C unter Schutzgas gerührt. Zur Aufarbeitung wird in 40 g Eis/40 ml konz. wässriger Ammoniaklösung gegossen und mit Methylenchlorid extrahiert. Die organischen Phasen werden mit Wasser neutral gewaschen, über Natriumsulfat getrocknet, filtriert und eingeengt. Der Rückstand wird mit n-Hexan/Essigester an Kieselgel chromatographiert. Es ergeben sich 1,58 g
17β-Hydroxy-17α-(3-hydroxyprop-1(Z)-enyl)-11β-(4-methylmercaptophenyl)-4,9(10)-estradien-3-on
als blassgelber fester Schaum.

$^1$H-NMR ($CDCl_3$): δ = 7,03 ppm (s, 4H, arom. CH)
5,8–5,5 (m, 2H, –CH=CH–,
$J_{cis}$ = 12 Hz)
2,35 (s, 3H, $-SCH_3$)

**Patentansprüche für die Vertragsstaaten BE, CH, LI, DE, FR, GB, IT, LU, NL, SE**

1. 11β-Aryl-Estradiene der allgemeinen Formel I

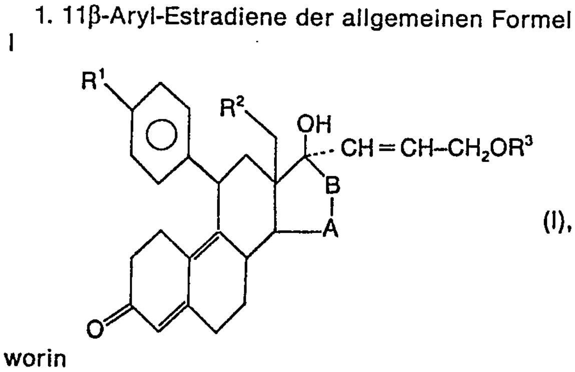

(I),

worin
$R^1$

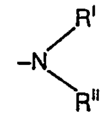

mit R' und R'' in der Bedeutung von Wasserstoff, Alkyl mit 1 bis 4 Kohlenstoffatomen oder Acyl mit 1 bis 8 Kohlenstoffatomen oder R' und R'' unter Einschluss von N in der Bedeutung eines 5- oder 6gliedrigen Ringes, wobei im Ring ausser N noch ein weiteres Heteroatom wie O, N, S enthalten sein kann, sowie die entsprechenden

N-Oxide und Säureadditionssalze oder $R^I$ und $R^{II}$ unter Einschluss von N in der Bedeutung eines gegebenenfalls durch Methyl substituierten Pyrrolringes,

$-OR^{III}$ mit $R^{III}$ in der Bedeutung von Wasserstoff, Methyl, Ethyl, Propyl, Methoxyphenyl, Allyl oder Dimethylaminoethyl,

$-SR^{IV}$ mit $R^{IV}$ in der Bedeutung von Wasserstoff, Methyl, Ethyl, Propyl oder Dimethylaminoethyl und

$R^2$ ein Wasserstoffatom, eine Methyl- oder Ethylgruppe,

$R^3$ ein Wasserstoffatom oder eine Acylgruppe mit 1 bis 8 Kohlenstoffatomen und

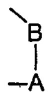 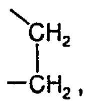, 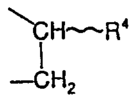, 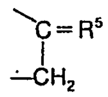, 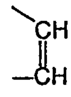, 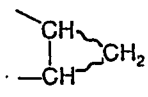,

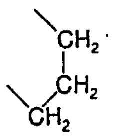, 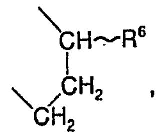, 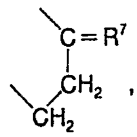, 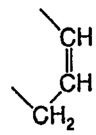 oder 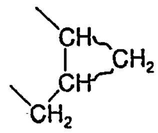

bedeuten, wobei $R^4$ und $R^6$ jeweils Alkyl mit 1 bis 4 Kohlenstoffatomen in α- oder β-Stellung und $R^5$ und $R^7$ jeweils eine E- oder Z-konfigurierte Alkylidengruppe darstellen und die $C_{20}/C_{21}$-Doppelbindung eine Z- oder E-Konfiguration besitzt.

2. 11β-(4-Dimethylaminophenyl)-17β-hydroxy-17α-(3-hydroxyprop-1(Z)-enyl)-4,9(10)-estradien-3-on.

3. 11β-(4-Dimethylaminophenyl)-17β-hydroxy-17α-(3-hydroxyprop-1(E)-enyl)-4,9(10)-estradien-3-on.

4. 17β-Hydroxy-17α-(3-hydroxyprop-1(Z)-enyl)-11β-(4-methylaminophenyl)-4,9(10)-estradien-3-on.

5. 11β-(4-Aminophenyl)-17β-hydroxy-17α-(3-hydroxyprop-1(Z)-enyl)-4,9(10)-estradien-3-on.

6. 11β-(4-Dimethylaminophenyl)-17β-hydroxy-17α-(3-hydroxyprop-1(Z)-enyl)-16β-methyl-4,9(10)-estradien-3-on.

7. 11β-(4-Dimethylaminophenyl)-17β-hydroxy-17α-(3-hydroxyprop-1(Z)-enyl)-16α-methyl-4,9(10)-estradien-3-on.

8. 17β-Hydroxy-17α-(3-hydroxyprop-1(Z)-enyl)-11β-[4-(piperidin-1-yl)phenyl]-4,9(10)-estradien-3-on.

9. 17β-Hydroxy-17α-(3-hydroxyprop-1(Z)-enyl)-11β-[4-(pyrrolidin-1-yl)phenyl]-4,9(10)-estradien-3-on.

10. 11β-[4-(2,5-Dimethylpyrrol-1-yl)phenyl]-17β-hydroxy-17α-(3-hydroxyprop-1(Z)-enyl)-4,9(10)-estradien-3-on.

11. 11β-(4-Dimethylaminophenyl)-16(E)-ethyliden-17β-hydroxy-17α-(3-hydroxyprop-1(Z)-enyl)-4,9(10)-estradien-3-on.

12. 17α-(3-Acetoxyprop-1(Z)-enyl)-11β-(4-dimethylaminophenyl)-17β-hydroxy-4,9(10)-estradien-3-on.

13. 11β-[(4-N-Formyl)-N-methylaminophenyl]-17β-hydroxy-17α-(3-hydroxyprop-1(Z)-enyl)-4,9(10)-estradien-3-on.

14. 11β-(4-N-Acetyl-N-methylaminophenyl)-17β-hydroxy-17α-(3-hydroxyprop-1(Z)-enyl-4,9(10)-estradien-3-on.

15. 11β-(4-Formylaminophenyl)-17β-hydroxy-17α-(3-hydroxyprop-1(Z)-enyl)-4,9(10)-estradien-3-on.

16. 11β-(4-Acetylaminophenyl)-17β-hydroxy-17α-(3-hydroxyprop-1(Z)-enyl)-4,9(10)-estradien-3-on.

17. 11β-(4-Dimethylaminophenyl)-17aβ-hydroxy-17aα-(3-hydroxyprop-1(Z)-enyl-D-homo-4,9,16-estratrien-3-on.

18. 17β-Hydroxy-17α-(3-hydroxyprop-1(Z)-enyl)-11β-4-methoxyphenyl)-4,9(10)-estradien-3-on.

19. 17β-Hydroxy-17α-(3-hydroxyprop-1(Z)-enyl)-11β-(4-methylmercaptophenyl)-4,9(10)-estradien-3-on.

20. Pharmazeutische Präparate, gekennzeichnet durch den Gehalt an Verbindungen gemäss Anspruch 1 bis 19.

21. Verfahren zur Herstellung von Verbindungen der allgemeinen Formel I

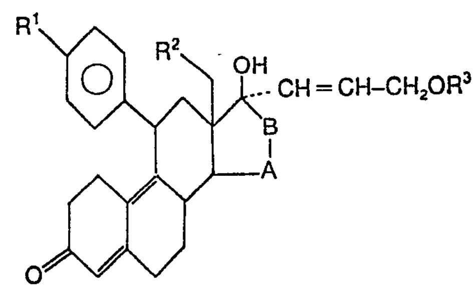

(I),

worin $R^1$, $R^2$, $R^3$ und

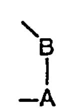

die in Anspruch 1 angegebene Bedeutung haben und die $C_{20}/C_{21}$-Doppelbindung eine Z- oder E-Konfiguration besitzt, dadurch gekennzeichnet, dass man in einer Verbindung der allgemeinen Formel II

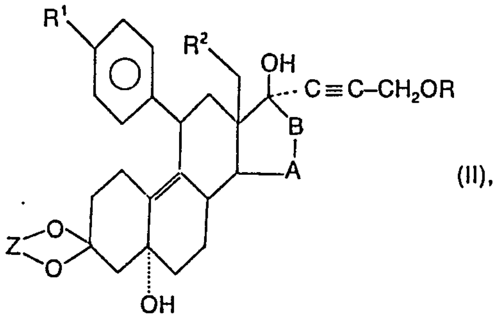

(II),

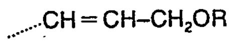

(III),

worin

$R^1$, $R^2$ und

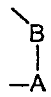

die in Formel I angegebene Bedeutung haben,

Z eine Ethylen- oder 2,2-Dimethyltrimethylengruppe und

R ein Wasserstoffatom oder eine im sauren Milieu oder hydrogenolytisch leicht abspaltbare organische Gruppe darstellt,

in Gegenwart eines desaktivierten Edelmetallkatalysators die acetylenische Dreifachbindung zur Z-konfigurierten $C_{20}/C_{21}$-Doppelbindung hydriert oder

in an sich bekannter Weise die acetylenische Dreifachbindung zur E-konfigurierten $C_{20}/C_{21}$-Doppelbindung reduziert

und anschliessend durch Einwirkung von verdünnter Säure, eines Pyridiniumsalzes einer starken Säure oder eines sauren Ionenaustauschers die Spaltung des 3-Ketalschutzes, die Entfernung einer gegebenenfalls vorhandenen mit Säure abspaltbaren Schutzgruppe R und die Wasserabspaltung unter Ausbildung des 4,9(10)-Dien-3-on-Systems bewirkt, und gegebenenfalls anschliessend eine freie 22-Hydroxygruppe acyliert.

22. Verfahren nach Anspruch 21, dadurch gekennzeichnet, dass man als desaktivierten Edelmetallkatalysator Palladium auf Bariumsulfat in Gegenwart eines tertiären Amins verwendet.

23. Verfahren nach Anspruch 21, dadurch gekennzeichnet, dass man als desaktivierten Edelmetallkatalysator Palladium auf Calciumcarbonat in Gegenwart von Pb $(OAcetyl)_2$ verwendet.

24. Verfahren nach Anspruch 21, dadurch gekennzeichnet, dass man Lithiumaluminiumhydrid als Reduktionsmittel verwendet.

25. Verwendung einer oder mehrerer Verbindung(en) gemäss den Ansprüchen 1 bis 19 zur Herstellung von Arzneimitteln.

**Patentansprüche für den Vertragsstaat AT**

1. Verfahren zur Herstellung von 11β-Aryl-Estradienen der allgemeinen Formel I

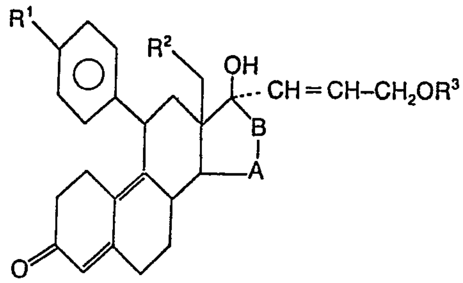

(I),

worin

$R^1$

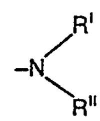

mit $R^I$ und $R^{II}$ in der Bedeutung von Wasserstoff, Alkyl mit 1 bis 4 Kohlenstoffatomen oder Acyl mit 1 bis 8 Kohlenstoffatomen oder $R^I$ und $R^{II}$ unter Einschluss von N in der Bedeutung eines 5- oder 6gliedrigen Ringes, wobei im Ring ausser N noch ein weiteres Heteroatom wie O, N, S enthalten sein kann, sowie die entsprechenden N-Oxide und Säureadditionssalze oder $R^I$ und $R^{II}$ unter Einschluss von N in der Bedeutung eines gegebenenfalls durch Methyl substituierten Pyrrolringes,

$-OR^{III}$ mit $R^{III}$ in der Bedeutung von Wasserstoff, Methyl, Ethyl, Propyl, Methoxyphenyl, Allyl oder Dimethylaminoethyl,

$-SR^{IV}$ mit $R^{IV}$ in der Bedeutung von Wasserstoff, Methyl, Ethyl, Propyl oder Dimethylaminoethyl und

$R^2$ ein Wasserstoffatom, eine Methyl- oder Ethylgruppe,

$R^3$ ein Wasserstoffatom oder eine Acylgruppe mit 1 bis 8 Kohlenstoffatomen und

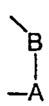 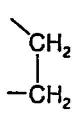, 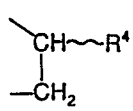, 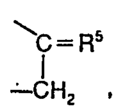, 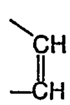, 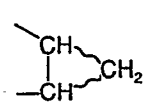,

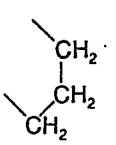, 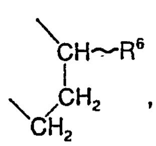, 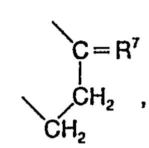, 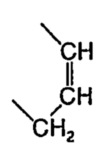 oder 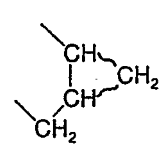

bedeuten, wobei $R^4$ und $R^6$ jeweils Alkyl mit 1 bis 4 Kohlenstoffatomen in α- oder β-Stellung und $R^5$ und $R^7$ jeweils eine E- oder Z-konfigurierte Alkylidengruppe darstellen und die $C_{20}/C_{21}$-Doppelbin-

dung eine Z- oder E-Konfiguration besitzt, dadurch gekennzeichnet, dass man in einer Verbindung der allgemeinen Formel II

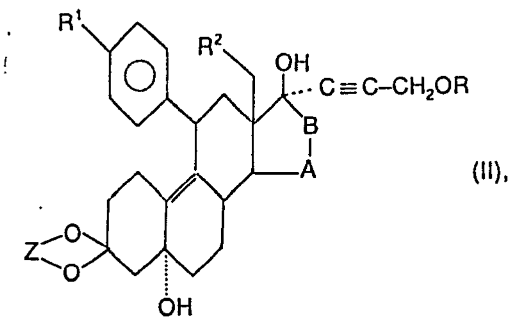

(II),

.....$CH=CH-CH_2OR$ (III),

worin

$R^1$, $R^2$ und

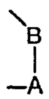

die in Formel I angegebene Bedeutung haben,

Z eine Ethylen- oder 2,2-Dimethyltrimethylengruppe und

R ein Wasserstoffatom oder eine im sauren Milieu oder hydrogenolytisch leicht abspaltbare organische Gruppe darstellt,

in Gegenwart eines desaktivierten Edelmetallkatalysators die acetylenische Dreifachbindung zur Z-konfigurierten $C_{20}/C_{21}$-Doppelbindung hydriert oder

in an sich bekannter Weise die acetylenische Dreifachbindung zur E-konfigurierten $C_{20}/C_{21}$-Doppelbindung reduziert,

und anschliessend durch Einwirkung von verdünnter Säure, eines Pyridiniumsalzes einer starken Säure oder eines sauren Ionenaustauschers die Spaltung des 3-Ketalschutzes, die Entfernung einer gegebenenfalls vorhandenen mit Säure abspaltbaren Schutzgruppe R und die Wasserabspaltung unter Ausbildung des 4,9(10)-Dien-3-on-Systems bewirkt, und gegebenenfalls anschliessend eine freie 22-Hydroxygruppe acyliert.

2. Verfahren nach Anspruch 1, dadurch gekennzeichnet, dass man als desaktivierten Edelmetallkatalysator Palladium auf Bariumsulfat in Gegenwart eines tertiären Amins verwendet.

3. Verfahren nach Anspruch 1, dadurch gekennzeichnet, dass man als desaktivierten Edelmetallkatalysator Palladium auf Calciumcarbonat in Gegenwart von Pb $(OAcetyl)_2$ verwendet.

4. Verfahren nach Anspruch 1, dadurch gekennzeichnet, dass man Lithiumaluminiumhydrid als Reduktionsmittel verwendet.

5. Verwendung einer oder mehrerer gemäss den Ansprüchen 1 bis 4 hergestellten Verbindung(en) zur Herstellung von Arzneimitteln.

**Claims for the contracting states BE, CH, LI, DE, FR, GB, IT, LU, NL, SE**

1. 11β-Aryloestradienes of the general formula I

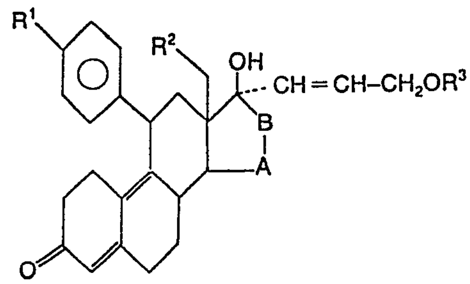

(I),

wherein

$R^1$ is

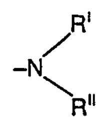

in which $R^I$ and $R^{II}$ represent hydrogen, alkyl having from 1 to 4 carbon atoms, or acyl having from 1 to 8 carbon atoms, or $R^I$ and $R^{II}$ with the inclusion of N represent a 5- or 6-membered ring wherein in addition to N another hetero atom, such as O, N or S, can be contained in the ring, as well as the corresponding N-oxides and acid addition salts, or $R^I$ and $R^{II}$ with the inclusion of N represent a pyrrole ring optionally substituted by methyl,

$-OR^{III}$ in which $R^{III}$ represents hydrogen, methyl, ethyl, propyl, methoxyphenyl, allyl or dimethylaminoethyl, or

$-SR^{IV}$ in which $R^{IV}$ represents hydrogen, methyl, ethyl, propyl or dimethylaminoethyl, and

$R^2$ is a hydrogen atom, a methyl group, or an ethyl group,

$R^3$ is a hydrogen atom or an acyl group having from 1 to 8 carbon atoms, and

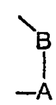

represents

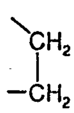, 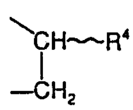, 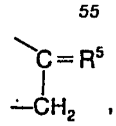, 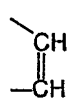, 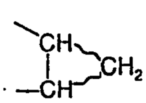,

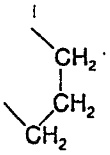, 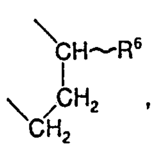, 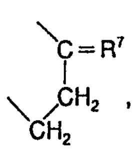, 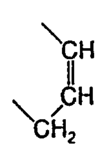 or 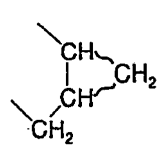

wherein $R^4$ and $R^6$ each represents alkyl having from 1 to 4 carbon atoms in the α- or β-configuration and $R^5$ and $R^7$ each represents an alkylidene group in the E- or Z-configuration, and the $C_{20}/C_{21}$-double bond has a Z- or E-configuration.

2. 11β-(4-Dimethylaminophenyl)-17β-hydroxy-17α-(3-hydroxyprop-1(Z)-enyl)-4,9(10)-oestradien-3-one.

3. 11β-(4-Dimethylaminophenyl)-17β-hydroxy-17α-(3-hydroxyprop-1(E)-enyl)-4,9(10)-oestradien-3-one.

4. 17β-Hydroxy-17α-(3-hydroxyprop-1(Z)-enyl)-11β-(4-methylaminophenyl)-4,9(10)-oestradien-3-one.

5. 11β-(4-Aminophenyl)-17β-hydroxy-17α-(3-hydroxyprop-1(Z)-enyl)-4,9(10)-oestradien-3-one.

6. 11β-(4-Dimethylaminophenyl)-17β-hydroxy-17α-(3-hydroxyprop-1(Z)-enyl)-16β-methyl-4,9(10)-oestradien-3-one.

7. 11β-(4-Dimethylaminophenyl)-17β-hydroxy-17α-(3-hydroxyprop-1(Z)-enyl)-16α-methyl-4,9(10)-oestradien-3-one.

8. 17β-Hydroxy-17α-(3-hydroxyprop-1(Z)-enyl)-11β-[4-(piperidin-2-yl)phenyl]-4,9(10)-oestradien-3-one.

9. 17β-Hydroxy-17α-(3-hydroxyprop-1(Z)-enyl)-11β-[4-(pyrrolidin-1-yl)phenyl]-4,9(10)-oestradien-3-one.

10. 11β-[4-(2,5-Dimethylpyrrol-1-yl)phenyl]-17β-hydroxy-17α-(3-hydroxyprop-1(Z)-enyl)-4,9(10)-oestradien-3-one.

11. 11β-(4-Dimethylaminophenyl)-16(E)-ethylidene-17β-hydroxy-17α-(3-hydroxyprop-1(Z)-enyl)-4,9(10)-oestradien-3-one.

12. 17α-(3-Acetoxyprop-1(Z)-enyl)-11β-(4-dimethylaminophenyl)-17β-hydroxy-4,9(10)-oestradien-3-one.

13. 11β-[(4-N-Formyl)-N-methylaminophenyl]-17β-hydroxy-17α-(3-hydroxyprop-1(Z)-enyl)-4,9(10)-oestradien-3-one.

14. 11β-(4-N-Acetyl-N-methylaminophenyl)-17β-hydroxy-17α-(3-hydroxyprop-1(Z)-enyl)-4,9(10)-oestradien-3-one.

15. 11β-(4-Formylaminophenyl)-17β-hydroxy-17α-(3-hydroxyprop-1(Z)-enyl)-4,9(10)-oestradien-3-one.

16. 11β-(4-Acetylaminophenyl)-17β-hydroxy-17α-(3-hydroxyprop-1(Z)-enyl)-4,9(10)-oestradien-3-one.

17. 11β-(4-Dimethylaminophenyl)-17aβ-hydroxy-17aα-(3-hydroxyprop-1(Z)-enyl)-D-homo-4,9,16-oestratrien-3-one.

18. 17β-Hydroxy-17α-(3-hydroxyprop-1(Z)-enyl)-11β-(4-methoxyphenyl)-4,9(10)-oestradien-3-one.

19. 17β-Hydroxy-17α-(3-hydroxyprop-1(Z)-enyl)-11β-(4-methylmercaptophenyl)-4,9(10)-oestradien-3-one.

20. Pharmaceutical preparations characterized in that they contain compounds according to claims 1–19.

21. Process for the preparation of compounds of the general formula I

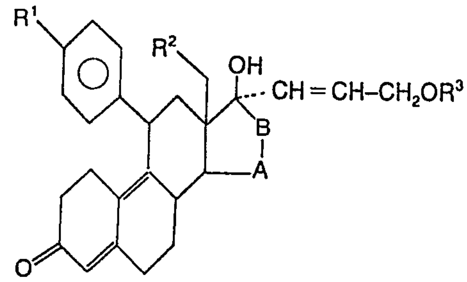

(I),

wherein $R^1$, $R^2$, $R^3$ and

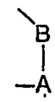

have the meanings given in claim 1 and the $C_{20}/C_{21}$-double bond has a Z- or E-configuration, characterized in that, in a compound of the general formula II

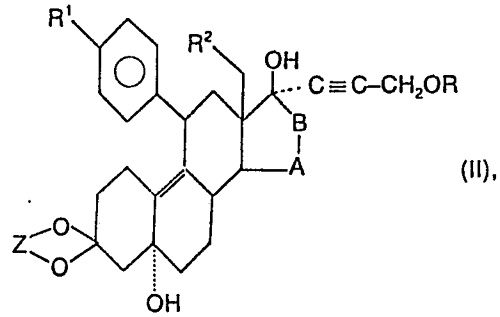

(II),

$......CH=CH-CH_2OR$ (III),

wherein

$R^1$, $R^2$ and

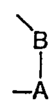

have the meanings given in formula I,

Z is an ethylene group or a 2,2-dimethyltrimethylene group and

R is a hydrogen atom or an organic group that can be readily split off in an acidic medium or by hydrogenolysis,

the acetylenic triple bond is hydrogenated to the Z-configured $C_{20}/C_{21}$-double bond in the presence of a deactivated noble metal catalyst,

or

the acetylenic triple bond is reduced in a manner known per se to the E-configured $C_{20}/C_{21}$-double bond,

and subsequently, by the action of a dilute acid, a pyridinium salt of a strong acid, or an acidic ion exchanger, the 3-ketal protection is cleaved, the removal of any protecting group R that can be split off with an acid is effected, and water is split off to form the 4,9(10)-dien-3-one system, and optionally thereafter a free 22-hydroxy group is acylated.

22. Process according to claim 21, characterized in that the deactivated noble metal catalyst used is palladium on barium sulfate in the presence of a tertiary amine.

23. Process according to claim 21, characterized in that the deactivated noble metal catalyst used is palladium on calcium carbonate in the presence of Pb (Oacetyl)$_2$.

24. Process according to claim 21, characterized in that lithium aluminium hydride is used as the reducing agent.

25. Use of one or more compound(s) according to claims 1 to 19 for the preparation of medicaments.

**Claims for the contracting state AT**

1. Process for the preparation of 11β-aryloestradienes of the general formula I

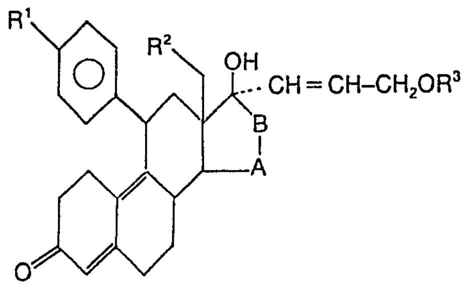

(I),

wherein

$R^1$ is

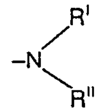

in which $R^I$ and $R^{II}$ represent hydrogen, alkyl having from 1 to 4 carbon atoms, or acyl having from 1 to 8 carbon atoms, or $R^I$ and $R^{II}$ with the inclusion of N represent a 5- or 6-membered ring wherein in addition to N another hetero atom, such as O, N or S, can be contained in the ring, as well as the corresponding N-oxides and acid addition salts, or $R^I$ and $R^{II}$ with the inclusion of N represent a pyrrole ring optionally substituted by methyl,

$-OR^{III}$ in which $R^{III}$ represents hydrogen, methyl, ethyl, propyl, methoxyphenyl, allyl or dimethylaminoethyl, or

$-SR^{IV}$ in which $R^{IV}$ represents hydrogen, methyl, ethyl, propyl or dimethylaminoethyl, and

$R^2$ is a hydrogen atom, a methyl group, or an ethyl group,

$R^3$ is a hydrogen atom or an acyl group having from 1 to 8 carbon atoms, and

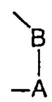

represents

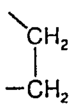, 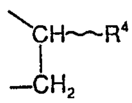 , 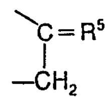 , 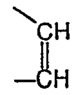, 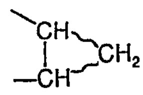 ,

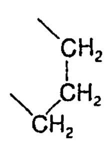 , 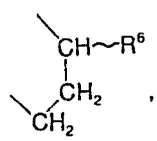 , 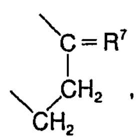 , 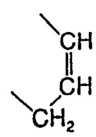 or 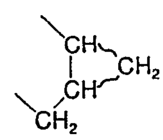

wherein $R^4$ and $R^6$ each represents alkyl having from 1 to 4 carbon atoms in the α- or β-configuration and $R^5$ and $R^7$ each represents an alkylidene group in the E- or Z-configuration, and the $C_{20}/C_{21}$-double bond has a Z- or E-configuration, characterized in that, in a compound of the general formula II

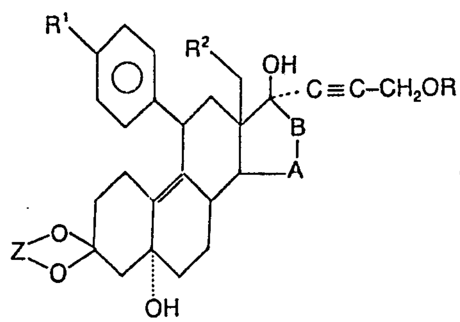

(II),

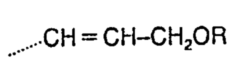

(III),

wherein

$R^1$, $R^2$ and

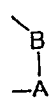

have the meanings given in formula I,

Z is an ethylene group or a 2,2-dimethyltrimethylene group and

R is a hydrogen atom or an organic group that can be readily split off in an acidic medium or by hydrogenolysis,

the acetylenic triple bond is hydrogenated to the Z-configured $C_{20}/C_{21}$-double bond in the presence of a deactivated noble metal catalyst, or

the acetylenic triple bond is reduced in a manner known per se to the E-configured $C_{20}/C_{21}$-double bond,

and subsequently, by the action of a dilute acid, a pyridinium salt of a strong acid, or an acidic ion exchanger, the 3-ketal protection is cleaved, the removal of any protecting group R that can be split off with an acid is effected, and water is split off to form the 4,9(10)-dien-3-one system, and optionally thereafter a free 22-hydroxy group is acylated.

2. Process according to claim 1, characterized in that the deactivated noble metal catalyst used is palladium on barium sulfate in the presence of a tertiary amine.

3. Process according to claim 1, characterized in that the deactivated noble metal catalyst used is palladium on calcium carbonate in the presence of $Pb(Oacetyl)_2$.

4. Process according to claim 1, characterized in that lithium aluminium hydride is used as the reducing agent.

5. Use of one or more compound(s) produced in accordance with claims 1 to 4 for the preparation of medicaments.

**Revendications pour les Etats contractants BE, CH, DE, FR, GB, IT, LI, LU, NL, SE**

1. Aryl-11 œstradiènes de formule générale I

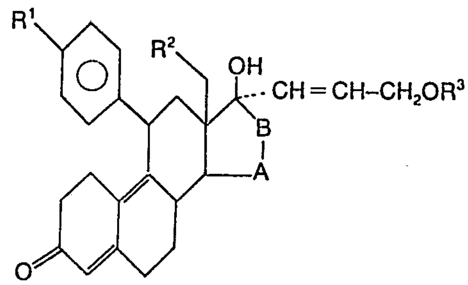

(I),

dans laquelle

$R^1$ représente

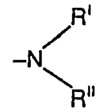

où $R^I$ et $R^{II}$ représentent chacun un atome d'hydrogène, un groupe alkyle ayant 1 à 4 atomes de carbone ou un groupe acyle ayant 1 à 8 atomes de carbone, ou bien $R^I$ et $R^{II}$ forment, avec inclusion de N, un noyau pentagonal ou hexagonal, un hétéroatome supplémentaire, comme O, N, S, pouvant être encore contenu en plus de N, ainsi que les N-oxydes et sels d'addition d'acides correspondants, ou bien $R^I$ et $R^{II}$ forment, avec inclusion de N, un noyau pyrrole éventuellement substitué par un groupe méthyle,

$-OR^{III}$, où $R^{III}$ représente un atome d'hydrogène, un groupe méthyle, éthyle, propyle, méthoxyphényle, allyle ou diméthylamino éthyle,

$-SR^{IV}$, où $R^{IV}$ représente un atome d'hydrogène, un groupe méthyle, éthyle, propyle ou diméthylamino éthyle, et

$R^2$ représente un atome d'hydrogène ou un groupe méthyle ou éthyle,

$R^3$ représente un atome d'hydrogène ou un groupe acyle ayant 1 à 8 atomes de carbone et

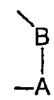

représente

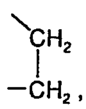, 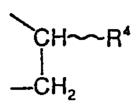, 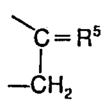, 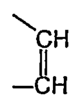, 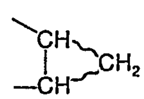,

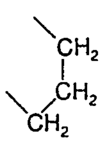, 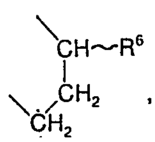, 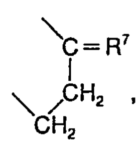, 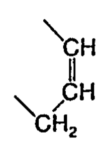 ou 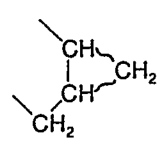

où $R^4$ et $R^6$ représentent chacun un groupe alkyle ayant 1 à 4 atomes de carbone en position α ou β, et $R^5$ et $R^7$ représentent chacun un groupe alkylidène de configuration E ou Z, et la double liaison en $C_{20}/C_{21}$ possède une configuration en Z ou en E.

2. La (diméthylamino-4 phényl)-11β hydroxy-17β (hydroxy-3 propényl-1(Z))-17α œstradiène-4,9(10) one-3.

3. La (diméthylamino-4 phényl)-11β hydroxy-17β (hydroxy-3 propényl-1(E))-17α œstradiène-4,9(10) one-3.

4. L'hydroxy-17β (hydroxy-3 propényl-1(Z))-17α (méthylamino-4 phényl-11β œstradiène-4,9(10) one-3.

5. L'(amino-4 phényl)-11β hydroxy-17β (hydroxy-3 propényl-1(Z))-17α œstradiène-4,9(10) one-3.

6. La (diméthylamino-4 phényl)-11β hydroxy-17β (hydroxy-3 propényl-1(Z))-17α méthyl-16β œstradiène-4,9(10) one-3.

7. La (diméthylamino-4 phényl)-11β hydroxy-17β (hydroxy-3 propényl-1(Z))-17α méthyl-16α œstradiène-4,9(10) one-3.

8. L'hydroxy-17β (hydroxy-3 propényl-1(Z))-17α [(pipéridinyl-1)-4 phényl]-11β œstradiène-4,9(10) one-3.

9. L'hydroxy-17β (hydroxy-3 propényl-1(Z))-17α [(pyrrolidinyl-1)-4 phényl]-11β œstradiène-4,9(10) one-3.

10. La [(diméthyl-2,5 pyrrolyl-1)-4 phényl]-11β hydroxy-17β (hydroxy-3 propényl-1(Z))-17α œstradiène-4,9(10) one-3.

11. La (diméthylamino-4 phényl)-11β éthylidène-16(E) hydroxy-17β (hydroxy-3 propényl-1(Z))-17α œstradiène-4,9(10) one-3.

12. L'(acétoxy-3 propényl-1(Z))-17α (diméthylamino-4 phényl)-11β hydroxy-17β œstradiène-4,9(10) one-3.

13. La [(N-formyl-4)N-méthylaminophényl]-11β hydroxy-17β (hydroxy-3 propényl-1(Z))-17α œstradiène-4,9(10) one-3.

14. La (N-acétyl-4 N-méthylaminophényl)-11β hydroxy-17β (hydroxy-3 propényl-1(Z))-17α œstradiène-4,9(10) one-3.

15. La (formylamino-4 phényl)-11β hydroxy-17β (hydroxy-3 propényl-1(Z))-17α œstradiène-4,9(10) one-3.

16. L'(acétylamino-4 phényl)-11β hydroxy-17β (hydroxy-3 propényl-1(Z))-17α œstradiène-4,9(10) one-3.

17. La (diméthylamino-4 phényl)-11β hydroxy-17aβ (hydroxy-3 propényl-1(Z))-17aα D-homo œstratriène-4,9,16 one-3.

18. L'hydroxy-17β (hydroxy-3 propényl-1(Z))-17α (méthoxy-4 phényl)-11β œstradiène-4,9(10) one-3.

19. L'hydroxy-17β (hydroxy-3 propényl-1(Z))-17α (méthylmercapto-4 phényl)-11β œstradiène-4,9(10)
one-3.

20. Préparations pharmaceutiques, caractérisées par une teneur en des composés selon la revendication 1 à 19.

21. Procédé pour préparer des composés de formule générale I

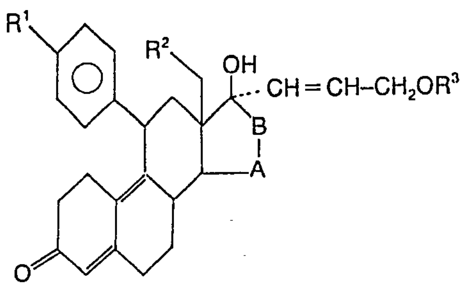

(I),

dans laquelle
$R_1$, $R_2$, $R_3$ et

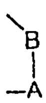

ont le sens indiqué à la revendication 1,
et la double liaison en $C_{20}/C_{21}$ possède une configuration en Z ou en E, procédé caractérisé en ce que, dans un composé de formule générale II

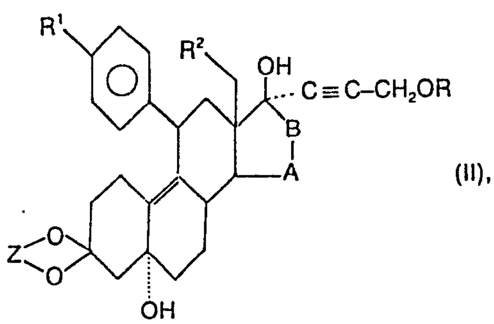

(II),

$\cdots CH=CH-CH_2OR$ (III),

dans laquelle
$R^1$, $R^2$ et

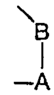

ont le sens indiqué pour la formule I,

Z représente un groupe éthylène ou diméthyl-2,2 triméthylène, et

R représente un atome d'hydrogène ou un groupe organique facilement séparable en milieu acide ou par hydrogénolyse,
on hydrogène la triple liaison acétylénique, en opérant en présence d'un catalyseur à base d'un métal noble désactivé, pour obtenir une double liaison en $C_{20}/C_{21}$ à configuration en Z,
ou
de façon connue en soi, on réduit la triple liaison acétylénique pour obtenir une double liaison en $C_{20}/C_{21}$ à configuration E,
et, par l'action d'un acide dilué, d'un sel de pyridinium d'un acide fort ou d'un échangeur d'ions acide, on provoque ensuite la séparation du groupe protecteur cétal-3, l'élimination d'un groupe protecteur R, éventuellement présent et séparable à l'aide d'un acide, et la séparation d'eau avec formation du système diène-4,9(10)one-3, et éventuellement ensuite on acyle un groupe hydroxy libre en 22.

22. Procédé selon la revendication 21, caractérisé en ce qu'on utilise, comme catalyseur à base de métal noble désactivé, du palladium sur du sulfate de baryum, en opérant en présence d'une amine tertiaire.

23. Procédé selon la revendication 21, caractérisé en ce qu'on utilise, comme catalyseur à base d'un métal noble désactivé, du palladium sur du carbonate de calcium en présence de Pb (O acétyle)$_2$.

24. Procédé selon la revendication 21, caractérisé en ce qu'on utilise comme réducteur de l'hydrure de lithium et d'aluminium.

25. Utilisation d'un ou plusieurs composé(s) selon les revendications 1 à 19 pour préparer des médicaments.

**Revendications pour l'Etat contractant AT**

1. Procédé pour préparer des aryl-11β œstradiènes de formule générale I

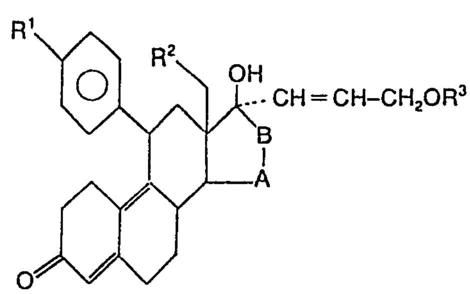

(I),

dans laquelle
$R^1$ représente

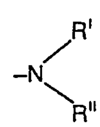

où $R^I$ et $R^{II}$ représentent chacun un atome d'hydrogène, un groupe alkyle ayant 1 à 4 atomes de carbone ou un groupe acyle ayant 1 à 8 atomes de carbone, ou bien $R^I$ et $R^{II}$, forment en comprenant N, un noyau pentagonal ou hexagonal, ce noyau pouvant contenir également, en plus de N, un autre hétéroatome comme O, N, S, ainsi que les N-oxydes et sels d'addition d'acides correspondants, ou bien $R^I$ et $R^{II}$ forment, en incorporant N, un noyau pyrrole éventuellement substitué par un groupe méthyle,

$-OR^{III}$, où $R^{III}$ représente un atome d'hydrogène, un groupe méthyle, éthyle, propyle, méthoxyphényle, allyle ou diméthylamino éthyle,

$-SR^{IV}$, $R^{IV}$ représentant un atome d'hydrogène, un groupe méthyle, éthyle, propyle ou diméthylamino éthyle, et

$R^2$ représente un atome d'hydrogène, un groupe méthyle ou éthyle,

$R^3$ représente un atome d'hydrogène ou un groupe acyle ayant 1 à 8 atomes de carbone, et

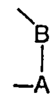

représente

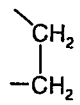, 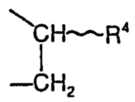, 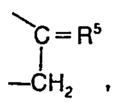, 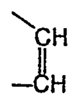, 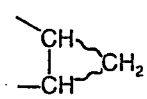,

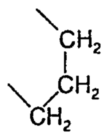, 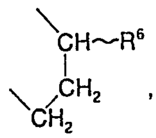, 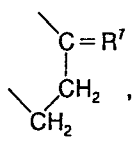, 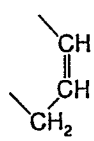 ou 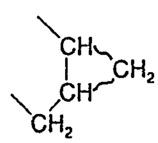

$R^4$ et $R^6$ représentant chacun un groupe alkyle ayant 1 à 4 atomes de carbone, en position α ou β, et $R^5$ et $R^7$ représentant chacun un groupe alkylidène de configuration en E ou Z, et la double liaison en $C_{20}/C_{21}$ possédant une configuration en Z ou en E,

procédé caractérisé en ce que, dans un composé de formule générale II

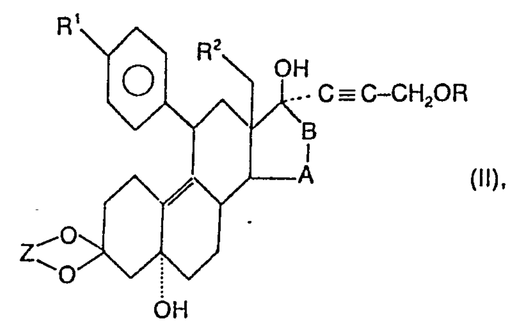

(II),

$$\cdots CH=CH-CH_2OR \qquad (III),$$

dans laquelle

$R^1$, $R^2$ et

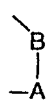

ont le sens indiqué à propos de la formule I,

Z représente un groupe éthylène ou 2,2-diméthyltriméthylène, et

R représente un atome d'hydrogène ou un groupe organique facilement séparable en milieu acide ou par hydrogénolyse, on hydrogène, en présence d'un catalyseur à base de métal noble désactivé, la triple liaison acétylénique pour obtenir une double liaison en $C_{20}/C_{21}$ en configuration Z, ou

on réduit de façon connue en soi la triple liaison acétylénique pour obtenir une double liaison en $C_{20}/C_{21}$ de configuration E,

puis, en faison agir de l'acide dilué, un sel de pyridinium d'un acide fort ou un échangeur d'ions acide, on provoque la séparation du groupe protecteur cétal-3, l'enlèvement d'un groupe protecteur R, séparable par de l'acide et éventuellement présent et la séparation d'eau, avec formation du système diène-4,9(10) one-3, et éventuellement ensuite on acyle un groupe hydroxyle libre en 22.

2. Procédé selon la revendication 1, caractérisé en ce qu'on utilise, comme catalyseur à base de métal noble désactivé, du palladium sur du sulfate de baryum, en opérant en présence d'une amine tertiaire.

3. Procédé selon la revendication 1, caractérisé en ce qu'on utilise, comme catalyseur à base de métal noble désactivé, du palladium sur du carbonate de calcium, en présence de Pb $(Oacétyle)_2$.

4. Procédé selon la revendication 1, caractérisé en ce qu'on utilise comme réducteur de l'hydrure de lithium et d'aluminium.

5. Utilisation d'un ou plusieurs composé(s), préparé(s) selon les revendications 1 à 4, pour préparer des médicaments.